(19) Europäisches Patentamt / European Patent Office / Office européen des brevets

(11) EP 3 481 416 B1

(12) EUROPEAN PATENT SPECIFICATION

(45) Date of publication and mention of the grant of the patent:
**09.09.2020 Bulletin 2020/37**

(21) Application number: **17742959.4**

(22) Date of filing: **07.07.2017**

(51) Int Cl.:
**A61K 38/48** (2006.01)     **A61K 38/38** (2006.01)
**A61P 7/04** (2006.01)

(86) International application number:
**PCT/EP2017/067127**

(87) International publication number:
**WO 2018/007601 (11.01.2018 Gazette 2018/02)**

(54) **SUBCUTANEOUS ADMINISTRATION OF LONG-ACTING FACTOR IX IN HUMANS**

SUBKUTANE APPLIKATION VON LANGLEBIGEM FACTOR IX IN MENSCHEN

ADMINISTRATION SOUS-CUTANÉE DE FACTEUR IX À ACTION PROLONGÉE CHEZ DES HUMAINS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.07.2016 US 201662359745 P**

(43) Date of publication of application:
**15.05.2019 Bulletin 2019/20**

(60) Divisional application:
**20165521.4**

(73) Proprietor: **CSL Behring Lengnau AG**
**2543 Lengnau BE (CH)**

(72) Inventors:
• **VOIGT, Christine**
  **Devon, Pennsylvania 19333 (US)**
• **PULI, Shilpa**
  **Belle Mead, New Jersey 08502 (US)**
• **ROBERTS, John**
  **Chester Springs, Pennsylvania 19425 (US)**
• **NOLTE, Marc**
  **35041 Marburg (DE)**

(74) Representative: **Hauser, Hans-Peter et al**
**CSL Behring GmbH**
**Patents & Licenses**
**Emil-von-Behring-Strasse 76**
**35041 Marburg (DE)**

(56) References cited:
**WO-A1-2014/052490     WO-A1-2015/085276**
**WO-A1-2015/095925**

• **JOHN SHEEHAN ET AL: "New developments in the management of moderate-to-severe hemophilia B", JOURNAL OF BLOOD MEDICINE, 1 April 2016 (2016-04-01), page 27, XP55410868, DOI: 10.2147/JBM.S81520**
• **MASSIMO MORFINI: "Pharmacokinetic drug evaluation of albutrepenonacog alfa (CSL654) for the treatment of hemophilia", EXPERT OPINION ON DRUG METABOLISM & TOXICOLOGY, vol. 12, no. 11, 19 November 2016 (2016-11-19), pages 1359-1365, XP055410481, GB ISSN: 1742-5255, DOI: 10.1080/17425255.2016.1240168**
• **Elena Santagostino ET AL: "Long-acting recombinant coagulation factor IX albumin fusion protein (rIX-FP) in hemophilia B: results of a phase 3 trial for the PROLONG-9FP Investigators Study Group Specialized Hospital for Active Treatment", , 11 January 2016 (2016-01-11), XP055410474, DOI: 10.1182/blood- Retrieved from the Internet: URL:http://www.bloodjournal.org/content/12 7/14/1761.full.pdf [retrieved on 2017-09-27]**

## Description

Field of the Invention

The present invention relates to clinically relevant dosing regimens of prophylactic subcutaneously administered long-acting factor IX (i.e., human FIX fused to human albumin) in human patients.

Background of the Invention

Hemophilia B is an X-linked recessive inherited bleeding disorder resulting from a deficiency of coagulation factor IX (FIX), a coagulation factor central to the process of blood coagulation. Signs and symptoms of hemophilia B are variable, depending on the severity of FIX deficiency and the location of bleeding. Most often, bleeding is characterized by spontaneous or trauma-induced hemorrhage into joints, muscles, and soft tissues. Recurrent bleeding in the same location may lead to permanent injury of the joint. Rare but life-threatening bleeding may also occur in the central nervous system, throat, or gastrointestinal tract.

Prophylaxis is currently considered as the optimal care for patients with severe hemophilia since it reduces the incidence of all bleeding episodes and when initiated at a young age, also reduces the development of arthropathy.

In the clinical investigation of the intravenous application route, long-acting human FIX fused to human albumin ("rIX-FP") has been shown to have a long half-life when administered intravenously, and the completed clinical studies show excellent safety and efficacy. Subjects on 7-day, 10-day or 14-day prophylaxis treatment with rIX-FP had a median annualized spontaneous bleeding rate (AsBR) of 0.00. In children < 12 years, the overall median AsBR was 0.00 bleeds per year per subject on weekly (7-day) prophylaxis. Bleeding episodes were successfully treated with 1 or 2 injections of rIX-FP; 98.6% of the bleeding episodes in the adult study (Study CSL654_3001) and 97.2% of the bleeding episodes in the pediatric study (CSL654_3002). This long-acting factor IX was a major improvement in hemophilia management, since it allows less frequent intravenous administrations to patients than FIX alone.

A pharmaceutical formulation comprising factor VIII or factor IX with an activity of at least 200 IU/ml and an additive increasing its bioavailability for subcutaneous administration and the use thereof for manufacturing of a medicament for treating hemophilia B is described in US5925739 A, but data is only provided for factor VIII. In WO2012/006624 therapeutic chimeric polypeptides comprising FIX are described, which can be fused to FcRn Binding Partners such as Fc. This document suggests administering at least about 10 IU/kg FIXFc, at an interval of about once weekly or longer, for prophylactic therapy. This document only discloses data for the FIXFc fusion protein, which is a different fusion protein to the one analyzed in the present invention, and it has different pharmacokinetic properties therefore requiring different dosing regimens to maintain certain trough levels of factor IX activity.

WO2014/052490 discloses a method of treatment for administering a long-acting Factor IX Fc fusion polypeptide to a human subject at a dose of about 10 IU/kg to about 200 IU/kg with a dosing interval of about once weekly or longer. WO2015/095925 discloses data on prophylactic intravenous dosing regimens of long-acting factor IX, including FIX linked to albumin ("rIX-FP"). Doses of 25-75 IU/kg are described as being administered at an interval of at least once a week. No data is disclosed in either of these documents regarding subcutaneous administration of long-acting FIX in humans.

Although Liles et al. published a report of dosing a single hemophilia B patient with a single subcutaneous dose of 9.3 U/kg plasma-derived FIX (Thromb Haemost 1997;77(5):944-8), there is very limited clinical data regarding subcutaneous administration of FIX to hemophilia B patients. Pre-clinical studies have shown dramatically different bioavailability data in various animal models. For example, the pre-clinical data in the present invention shows that the bioavailability of subcutaneously administered rIX-FP was about 13-50%, depending on the animal species.

A Phase I exploratory study of the PK of rIX-FP after subcutaneous administration in hemophilia B patients was conducted. The present invention shows for the first time, which subcutaneous dosing regimens of a long-acting FIX can be used for treating hemophilia B in human patients. Since subcutaneous injections can be easily administered either by patients themselves or, in the case of children, by their caregivers at home, these results allow significant improvement of hemophilia B management, since it allows more convenient and less painful injections of FIX. Subcutaneous administration also avoids the risk of contamination and clot formation in a venous access device, which may otherwise prevent some very young children with hemophilia from receiving adequate care.

Specifically, the present invention relates to a fusion protein comprising human FIX and human albumin ("rIX-FP"), wherein the human FIX is connected to the N-terminus of human albumin via a peptide linker cleavable by proteases involved in coagulation or activated by coagulation enzymes with the following exemplary limiting subcutaneous dosing regimens: a dose of 10-50 IU/kg at a dosing interval of once a day to once per week, preferably the dose is 25 IU/kg at a dosing interval of once a day, every 2 days or every 3 days. These subcutaneous dosing regimens have been shown to provide a sufficient bioavailability to maintain a clinically significant steady state level of FIX in hemophilic patients.

This advance is a significant further step in improving hemophilia B management, since infusions and intravenous injections can be completely avoided by allowing the patient to easily self-administer factor IX. Thus, the invention reduces the risk of infection due to possibly contaminated injection and infusion equipment, and increases patient compliance by avoiding the hospital and doctor visits required for intravenous injections and infusions. The invention also provides a therapeutic option for patients with venous access issues.

Summary of the Invention

The present invention provides clinically relevant dosing regimens for subcutaneous administration of rIX-FP. The dosing regimens achieve trough levels of FIX sufficient to prevent bleeding over the treatment period. The embodiments according to the present invention are described in the claims.

Specifically, the invention relates to a fusion protein comprising

a) human Factor IX (FIX), and
b) human albumin

for use in a method of preventing bleeding in a human patient in a prophylactic dosing regimen, wherein the human FIX is connected to the N-terminus of human albumin via a peptide linker cleavable by proteases involved in coagulation or activated by coagulation enzymes, and wherein the fusion protein is to be administered subcutaneously to the subject at a dose of about 10-50 IU/kg, using a dosing interval of about once a day to about once per week. The dose may be about 10-20 IU/kg, about 20-30 IU/kg, about 30-40 IU/kg, about 40-about 50 IU/kg, about 10 IU/kg, about 15 IU/kg, about 20 IU/kg, about 25 IU/kg, about 30 IU/kg, about 35 IU/kg, about 40 IU/kg, about 45 IU/kg or about 50 IU/kg. In a preferred embodiment, the dose is about 10 IU/kg. In another preferred embodiment, the dose is about 20 IU/kg. In a further preferred embodiment, the dose is about 25 IU/kg. In yet a further preferred embodiment, the dose is 50 IU/kg. In any one of these embodiments, the dosing interval may be about once a day, or about four times a week, or about every 2 days, or about every 3 days, or about twice a week, or about every 5 days, or about once per week.

In one preferred embodiment, the dose is about 10 IU/kg and the dosing interval is about once a day, or about four times a week, or about every 2 days, or about every 3 days or about twice a week.

In another preferred embodiment, the dose is 25 IU/kg and the dosing interval is about once a day, or about four times a week, or about every 2 days, or about every 3 days, or about twice a week, or about every 5 days, or about once per week.

In another preferred embodiment, the dose is 50 IU/kg and the dosing interval is about once a day, or about four times a week, or about every 2 days, or about every 3 days, or about twice a week, or about every 5 days, or about once per week.

In a highly preferred embodiment, the dose is 25 IU/kg and the dosing interval is about once a day. In another highly preferred embodiment, the dose is 25 IU/kg and the dosing interval is about every 2 days. In still a further highly preferred embodiment, the dose is 25 IU/kg and the dosing interval is about every 3 days.

In a highly preferred embodiment, the dose is about 25 IU/kg, the dosing interval is about once a day and the total FIX activity trough level in the plasma is maintained between about 8 to about 34 IU/dL. In another highly preferred embodiment, the dose is about 25 IU/kg, the dosing interval is about once a day and the total FIX activity trough level in the plasma is maintained between about 10 to about 30 IU/dL. In another highly preferred embodiment, the dose is about 25 IU/kg, the dosing interval is about once a day and the total FIX activity trough level in the plasma is maintained between about 15 to about 25 IU/dL. In still another highly preferred embodiment, the dose is about 25 IU/kg, the dosing interval is about once a day and the total FIX activity trough level in the plasma is maintained between about 18 to about 22 IU/dL. In another highly preferred embodiment, the dose is about 25 IU/kg, the dosing interval is about once a day and the total FIX activity trough level in the plasma is maintained at about 17 IU/dL.

In an alternative highly preferred embodiment, the dose is about 25 IU/kg, the dosing interval is about every 2 days and the total FIX activity trough level in the plasma is maintained between about 4 to about 17 IU/dL. In another highly preferred embodiment, the dose is about 25 IU/kg, the dosing interval is about every 2 days and the total FIX activity trough level in the plasma is maintained between about 6 to about 15 IU/dL. In still another highly preferred embodiment, the dose is about 25 IU/kg, the dosing interval is about every 2 days and the total FIX activity trough level in the plasma is maintained between about 7 to about 12 IU/dL. In another highly preferred embodiment, the dosing interval is about every 2 days and the total FIX activity trough level in the plasma is maintained at about 8 IU/dL.

In a further highly preferred embodiment, the dose is about 25 IU/kg, the dosing interval is about every 3 days and the total FIX activity trough level in the plasma is maintained between about 2.5 to about 12 IU/dL. In another highly preferred embodiment, the dose is about 25 IU/kg, the dosing interval is about every 3 days and the total FIX activity trough level in the plasma is maintained between about 3 to about 12 IU/dL. In another highly preferred embodiment, the dose is about 25 IU/kg, the dosing interval is about every 3 days and the total FIX activity trough level in the plasma is maintained between about 4 to about 11 IU/dL. In still another highly preferred embodiment, the dose is about 25 IU/kg, the dosing interval is about every 3 days and the total FIX activity trough level in the plasma is maintained between about 4.5 to about 10 IU/dL. In another highly preferred embodiment, the dose is about 25 IU/kg, the dosing interval is about every 3 days and the total FIX activity trough level in the plasma is maintained at about 5 IU/dL.

In preferred embodiments, the plasma level of the FIX maintains a trough level of at least about 1% above baseline for the entire dosing interval, preferably at least about 3% above baseline for the entire dosing interval, more preferably at least about 5% above baseline for the entire dosing interval, still more preferably at least about 10% above baseline for the entire dosing interval, and most preferably at least about 15% above baseline for the entire dosing interval.

In a preferred embodiment, the linker is cleavable by FIXa and/or by FVIIa/Tissue Factor (TF). In a particularly preferred embodiment, the linker comprises a sequence selected from SEQ ID NO: 1 and SEQ ID NO: 2.

SEQ ID NO: 1

Pro Val Ser Gln Thr Ser Lys Leu Thr Arg Ala Glu Thr Val Phe Pro Asp Val

1 5 10 15

SEQ ID NO: 2

Pro Ser Val Ser Gln Thr Ser Lys Leu Thr Arg Ala Glu Thr Val Phe Pro Asp Val

1 5 10 15

In an alternative embodiment, the linker is 90% identical to one of SEQ ID NO: 1 and SEQ ID NO: 2. In another embodiment, it is 80% identical to one of SEQ ID NO: 1 and SEQ ID NO: 2. In still another embodiment, it is 70% identical to one of SEQ ID NO: 1 and SEQ ID NO: 2. In still another embodiment, it is 60% identical to one of SEQ ID NO: 1 and SEQ ID NO: 2. In a further embodiment, it is 50% identical to one of SEQ ID NO: 1 and SEQ ID NO: 2.

Preferably, the fusion protein of the invention has at least 70% identity to the sequence set forth in SEQ ID NO: 3.

SEQ ID NO: 3 (mature rIX-FP)

FIX (aa 1-416)

Linker sequence (bold and underlined aa 416-433)

Albumin sequence (aa 434-1018)

| | | |
|---|---|---|
| 1 | YNSGKLEEFVQGNLERECMEEKCSFEEAREVFENTERTTEFWKQYVDGDQCESNPCLNGG | 60 |
| 61 | SCKDDINSYECWCPFGFEGKNCELDVTCNIKNGRCEQFCKNSADNKVVCSCTEGYRLAEN | 120 |
| 121 | QKSCEPAVPFPCGRVSVSQTSKLTRAETVFPDVDYVNSTEAETILDNITQSTQSFNDFTR | 180 |
| 181 | VVGGEDAKPGQFPWQVVLNGKVDAFCGGSIVNEKWIVTAAHCVETGVKITVVAGEHNIEE | 240 |
| 241 | TEHTEQKRNVIRIIPHHNYNAAINKYNHDIALLELDEPLVLNSYVTPICIADKEYTNIFL | 300 |
| 301 | KFGSGYVSGWGRVFHKGRSALVLQYLRVPLVDRATCLRSTKFTIYNNMFCAGFHEGGRDS | 360 |
| 361 | CQGDSGGPHVTEVEGTSFLTGIISWGEECAMKGKYGIYTKVSRYVNWIKEKTKLT**PVSQT** | 420 |
| 421 | **SKLTRAETVFPDV**DAHKSEVAHRFKDLGEENFKALVLIAFAQYLQQCPFEDHVKLVNEVT | 480 |
| 481 | EFAKTCVADESAENCDKSLHTLFGDKLCTVATLRETYGEMADCCAKQEPERNECFLQHKD | 540 |
| 541 | DNPNLPRLVRPEVDVMCTAFHDNEETFLKKYLYEIARRHPYFYAPELLFFAKRYKAAFTE | 600 |
| 601 | CCQAADKAACLLPKLDELRDEGKASSAKQRLKCASLQKFGERAFKAWAVARLSQRFPKAE | 660 |
| 661 | FAEVSKLVTDLTKVHTECCHGDLLECADDRADLAKYICENQDSISSKLKECCEKPLLEKS | 720 |
| 721 | HCIAEVENDEMPADLPSLAADFVESKDVCKNYAEAKDVFLGMFLYEYARRHPDYSVVLLL | 780 |
| 781 | RLAKTYETTLEKCCAAADPHECYAKVFDEFKPLVEEPQNLIKQNCELFEQLGEYKFQNAL | 840 |
| 841 | LVRYTKKVPQVSTPTLVEVSRNLGKVGSKCCKHPEAKRMPCAEDYLSVVLNQLCVLHEKT | 900 |
| 901 | PVSDRVTKCCTESLVNRRPCFSALEVDETYVPKEFNAETFTFHADICTLSEKERQIKKQT | 960 |
| 961 | ALVELVKHKPKATKEQLKAVMDDFAAFVEKCCKADDKETCFAEEGKKLVAASQAALGL | 1018 |

The sequence of the fusion protein may have at least 75% identity to the sequence set forth in SEQ ID NO: 3. The sequence of the fusion protein may have at least 80% percent identity to the sequence set forth in SEQ ID NO: 3. The sequence of the fusion protein may have at least 85% percent identity to the sequence set forth in SEQ ID NO: 3. The sequence of the fusion protein may have at least 90% percent identity to the sequence set forth in SEQ ID NO: 3. The sequence of the fusion protein may have at least 95% percent identity to the sequence set forth in SEQ ID NO: 3. The sequence of the fusion protein may have at least 98% percent identity to the sequence set forth in SEQ ID NO: 3. The sequence of the fusion protein may have at least 99% percent identity to the sequence set forth in SEQ ID NO: 3. In a more preferred embodiment, the sequence of the fusion protein has the sequence set forth in SEQ ID NO: 3.

In preferred embodiments of the invention, the human patient suffers from hemophilia B.

For any of the embodiments of the invention, the fusion protein is preferably provided for administration at a concentration of about 100 to 400 IU/ml, preferably about 100, 200 or 400 IU/ml. The fusion protein may also be provided for administration at concentrations of 600 IU/ml or 1200 IU/ml.

The invention also encompasses a fusion protein comprising a half-life prolonged FIX (with more than 3 times the terminal half-life of FIX) for use in a method of preventing bleeding in a human patient under treatment with intravenously administered FIX or intravenously administered half-life prolonged FIX, wherein the half-life prolonged FIX protein is to be administered subcutaneously to the subject. In another aspect, the invention encompasses a fusion protein comprising half-life prolonged FIX (with more than 3 times the terminal half-life of FIX) for use in a combination treatment of preventing bleeding in a human patient, wherein the patient is first treated with intravenous FIX or intravenously administered half-life prolonged FIX and the fusion protein is to be administered subcutaneously to the subject after the intravenous FIX treatment is initiated. Intravenous administration of half-life prolonged FIX is described in WO2015/095925 and WO2014/052490. Preferably, the fusion protein comprises a) Factor IX (FIX), and b) a half-life enhancing polypeptide (HLEP). In a highly preferred embodiment, the fusion protein is comprised of a) human Factor IX (FIX), and b) human albumin, wherein the human FIX is connected to the N-terminus of human albumin via a peptide linker cleavable by proteases involved in coagulation or activated by coagulation enzymes. The subcutaneous dose is preferably administered at a dose of about 10-50 IU/kg, using a dosing interval of about once a day to about once per week. The dose may be about 10-20 IU/kg, about 20-30 IU/kg, about 30-40 IU/kg, about 40-about 50 IU/kg, about 10 IU/kg, about 15 IU/kg, about 20 IU/kg, about 25 IU/kg, about 30 IU/kg, about 35 IU/kg, about 40 IU/kg, about 45 IU/kg or about 50 IU/kg. In a preferred embodiment, the dose is about 10 IU/kg. In another preferred embodiment, the dose is about 20 IU/kg. In a further preferred embodiment, the dose is about 25 IU/kg. In yet a further preferred embodiment, the dose is 50 IU/kg. In any one of these embodiments, the dosing interval may be about once a day, or about four times a week, or about every 2 days, or about every 3 days, or about twice a week, or about every 5 days, or about once per week. In highly preferred embodiments, the dose is 25 IU/kg and the dosing interval is about once a day, about once every 2 days, or about once every 3 days. Preferably, the method involves a prophylactic dosing regimen. In a another highly preferred embodiment, the fusion protein is comprised of a) human Factor IX (FIX), and b) the Fc part of an antibody or of a) human Factor IX and b) XTEN. In all of the above fusion proteins the human FIX and the HELP can be linked either directly or via a peptidic linker. In preferred embodiments the linker is cleavable by proteases, wherein the proteases are preferably proteases involved in coagulation or activated by coagulation enzymes. In another aspect, the invention encompasses a conjugated half-life prolonged human FIX (with more than 3 times the terminal half-life of FIX) for use in a combination treatment of preventing bleeding in a human patient, wherein the patient is first treated with intravenous FIX or intravenously administered conjugated half-life prolonged FIX and the conjugated half-life prolonged human FIX is to be administered subcutaneously to the subject after the intravenous FIX treatment is initiated. Conjugation leading to half-life prolongation comprises by way of nonlimiting examples pegylation or polysialylation. In a preferred embodiment the administration of the intravenously administered FIX or half-life prolonged FIX will be replaced by the subsequent subcutaneous administration of the half-life prolonged FIX. In another preferred embodiment the half-life prolonged FIX is administered intravenously and subcutaneously in parallel.

Description of the Figures

**Figure 1:** Plasma levels of human FIX antigen (rIX-FP) in rabbits (mean values ± SD, n=3/timepoint), linear scale.

**Figure 2:** Plasma levels of human FIX (rIX-FP) antigen in pigs (mean values ± SD, n=1-3/timepoint), linear scale.

**Figure 3:** Mean plasma concentrations of rIX-FP following intravenous and subcutaneous administration to cynomolgus monkeys at dose levels of 125 and 500 IU/kg.

**Figure 4:** Mean plasma concentrations of BeneFIX® and rIX-FP following subcutaneous administration to cynomolgus monkeys at a dose level of 500 IU/kg.

**Figure 5A:** Uncorrected FIX Activity vs Time plot by ID (Cohort 1-25 IU/kg) subcutaneous dose of rIX-FP in four different human patients (38, 39, 40 and 50).

**Figure 5B:** Uncorrected FIX Activity vs Time plot by ID (Cohort 2-50 IU/kg) subcutaneous dose of rIX-FP in five different human patients (38, 39, 16, 17 and 21).

**Figure 6A:** Corrected FIX Activity vs Time plot by ID (Cohort 1-25 IU/kg) subcutaneous dose of rIX-FP in four different human patients (38, 39, 40 and 50). The FIX activity levels were corrected for the exponential decline of the pre dose FIX levels. Once corrected, any negative values were set to 0.01.

**Figure 6B:** Corrected FIX Activity vs Time plot by ID (Cohort 2-50 IU/kg) subcutaneous dose of rIX-FP in five different human subjects (38 , 39 , 16 , 17 and 21). The FIX activity levels were corrected for the exponential decline of the pre dose FIX levels. Once corrected, any negative values were set to 0.01.

**Figure 7:** Cohort 3, 25 IU/kg every 3 days repeat dose Observed FIX activity vs Time plot by in three different human patients (38, 39, 40). Shaded in grey is the time interval between the first dose and the last repeat dose 15.

**Figure 8A:** Simulated plot of FIX Activity (IU/dL) vs Time for 25 IU/kg every day

**Figure 8B:** Simulated plot of FIX Activity (IU/dL) vs Time for 25 IU/kg every 2 days

**Figure 8C:** Simulated plot of FIX Activity (IU/dL) vs Time for 25 IU/kg every 3 days

**Figure 8D:** Simulated plot of FIX Activity (IU/dL) vs Time for 25 IU/kg every 3 days overlaid with observed PK data after the administration of the 15th dose from cohort 3 (n=3)

Detailed Description of the Invention

"Prophylactic treatment", "a prophylactic dosing regimen", "prevention of bleeding" or "preventing bleeding", as used herein, means administering a Factor IX fusion protein in multiple doses to a human patient over a course of time to increase the level of Factor IX activity in a patient's plasma. Preferably, the increased level is sufficient to decrease the incidence of spontaneous bleeding or to prevent bleeding in the event of an unforeseen injury. Prophylactic treatment decreases or prevents bleeding episodes, for example, those described under on-demand treatment. Prophylactic treatment may be fixed or may be individualized, as discussed under "dosing interval", e.g., to compensate for inter-patient variability.

"Dosing interval", as used herein, means the amount of time that elapses between multiple doses being administered to a human patient. The dosing interval in the methods of the invention using rIX-FP, may be at least about one and one-half to eight (1.5-8) times longer than the dosing interval required for an equivalent amount (in IU/kg) of said Factor IX without albumin (i.e., a polypeptide consisting of said FIX). The dosing interval when administering a Factor IX-albumin fusion protein of the invention may be at least about one and one-half times to eight (1.5-8) times longer than the dosing interval required for an equivalent amount of said Factor IX without albumin. The dosing interval may be at least about one and one-half to eight (1.5-8) times longer than the dosing interval required for an equivalent amount of said Factor IX without albumin (or a polypeptide consisting of said Factor IX).

"Median dose", as used herein, means half of the study human patients used higher than that dose and half of the study human patients used lower than that dose. "Mean dose" means an average dose (is computed by adding up all the doses and dividing by the total number of the doses). For a given dose, "about" means the dose indicated plus or minus 1, 2, 5, 10, 15 or 20% of that indicated dose. Alternatively, for a given dose, "about" means plus or minus 1 IU/kg. For a dosing interval of about once a day, "about" means plus or minus 6 hours. For a dosing interval of about four times a week or every two days, "about" means plus or minus 8 hours. For a dosing interval of about twice a week or every 3 days, "about" means plus or minus 12 hours. For a dosing interval of about every 5 days, "about" means plus or minus 15 hours. For a dosing interval of about once a week, "about" means plus or minus 18 hours.

"Total FIX activity trough level" means the plasma level trough level of FIX activity measured in IU/dL. This may be determined using direct coagulation assays like aPTT, PT, or the thrombin generation assays. However, other assays like, e.g., chromogenic assays applied for specific coagulation factors are also included. Examples for such assays or corresponding reagents are Pathromtin® SL (aPTT assay, Dade Behring) or Thromborel® S (Prothrombin time assay, Dade Behring) with corresponding coagulation factor deficient plasma (Dade Behring), Thrombin generation assay kits (Technoclone, Thrombinoscope) using e.g. coagulation factor deficient plasma, chromogenic assays like Biophen Factor IX (Hyphen BioMed), Staclot® FVIIa-rTF (Roche Diagnostics GmbH), Coatest® Factor VIII:C/4 (Chromogenix), or others. FIX activity is preferably measured by a one-stage aPTT assay with Pathromtin SL used as the activator. For total FIX activity trough levels, "about" means plus or minus 1 IU/dL.

"Baseline" means the FIX activity level in a given patient preferentially expressed in IU/dL or in % of the FIX activity in a healthy person which is defined to be 100 IU/dL or 100%. In severe hemophilia B the baseline level of a given patient is very low to zero or almost zero, whereas in moderate hemophilia the patient's baseline may be higher such as above 1%, above 2% or above 3% or above 4%, whereas in mild hemophilia the patient's baseline may be above 5% of the FIX activity concentration in a healthy person. When the fusion protein comprising i) human Factor IX (FIX) portion, and ii) human albumin is administered according to the present invention first the FIX activity concentration sharply increases and is the slowly cleared i.e. it is returning to the individual baseline level. In the present study baseline levels were higher due to the residual FIX levels from prior IV dosing which were corrected by subtracting the exponential decline of the pre-dose levels in order to arrive at the anticipated endogenous levels.

The "trough level" is the lowest level of said FIX biological activity throughout the dosing regimen during the treatment of a patient in need of FIX. Due to patient inter-variability, the trough level generally refers to median values, which means that half of the study patients had a higher trough level and half of the study patients had a lower trough level. Though using the median value is more common, the trough level from the PK data could also be calculated as a mean value, which has been determined by adding up the values for all patients and dividing by the number of patients. The trough level is preferably above 1%, more preferably above 2%, still more preferably above 3%, still more preferably above 4%, still more preferably above 5%, still more preferably above 6%, still more preferably above 7%, still more preferably above 8%, still more preferably above 9%, still more preferably above 10%, still more preferably above 11%, still more preferably above 12%, still more preferably above 13%, still more preferably above 14%, even more preferably above 15%. Further preferred trough levels are above 20%, above 25%, above 30%, above 35%, above 40%, above 45%, above 50%, above 55%, above 60%, above 65%, above 70%, above 75%, above 80%, above 85%, above 90%, above 95%.

"Maintaining the plasma level of total FIX activity at a trough of at least "about" a certain concentration means, that the total FIX activity in plasma will not fall below said concentration during a certain dosing regimen of a patient in need of FIX, wherein the total FIX activity concentration in normal human plasma is 1 IU/ml or 100 IU/dL, and wherein the FIX activity preferably is determined using a validated one-stage clotting method. FIX activity is the FIX biological activity.

"Maintaining the plasma level of FIX at a trough between about" a certain first concentration "and about" another certain second concentration means, that the FIX activity in plasma will not fall below said first concentration during a certain dosing regimen of a patient in need of FIX, and will not rise above said second concentration wherein the FIX activity concentration in normal human plasma is 1 IU/ml or 100 IU/dL, and wherein the FIX activity preferably is determined using a validated one-stage clotting method. FIX activity is the FIX biological activity.

"About" a certain trough level as used herein, means an average trough level. For a given trough level, "about" means the trough level indicated plus or minus 1, 2, 5, 10, 15, 20 or 30 % of that indicated trough level. Alternatively, for a given trough level, "about" means plus or minus 1 IU/dL.

Especially in severe hemophilia B, care has to be taken that the FIX activity concentration does not fall below a minimal level in order to prevent bleeding. This minimal level is called the desired trough level which consists of the endogenous level plus the lowest additional level of the administered FIX. In a severe hemophilia B patient when the baseline is practically zero a trough level of 1% above baseline means a FIX activity concentration of about 1% of the FIX activity concentration in a healthy person. In a moderate hemophilia B patient having a baseline level of 3% FIX activity of the FIX activity concentration in a healthy person a trough level of 1% above baseline means a FIX activity concentration of about 4% of the FIX activity concentration in a healthy person.

In the invention, the dosing interval may be about once a day, about four times a week, about every 2 days, about every 3 days, about twice a week, about every 5 days, or about once a week. This means that the interval may be about once every 1 to 7 days, including about every other day, about 5 times a week, about once every 2-4 days, about 3 times a week, about once every 4-6 days, about once every 5 days, about once every 6-8 days, about once every 7 days. In particular, dosing intervals of about once a day, about four times a week, about every 2 days, about every 3 days, about twice a week, about every 5 days, and about once a week are contemplated. The most preferred dosing intervals are about once a day, once every 2 days and once every 3 days.

The dosing interval may, alternatively, be an individualized interval that is determined for each patient based on pharmacokinetic data or other information about that patient. The individualized dose/dosing interval combination may be the same as those for fixed interval regimens in the preceding paragraphs, or may differ. The regimen may initially be at a fixed dosing interval, and then it may change to an individualized dosing interval. Alternatively, the regimen may initially be at a fixed dose (IU/kg) and dosing interval, and then it may change to an individualized dosing interval with the fixed dose. The regimen may also initially be at a fixed dosing interval and dose (IU/kg), and then it may change to an individualized dose with the same fixed dosing interval.

For a dosing schedule of about once a day, about 4 times a week, about every 2 days, about every 3 days, and about twice a week, the therapeutic doses of about 10-15 IU/kg, about 15-20 IU/kg, about 10 IU/kg, about 15 IU/kg, and about 20 IU/kg are envisioned.

Regarding dosing schedules of about once a day, 4 times a week, or about every 2 days, or about every 3 days, about twice a week, or about every 5 days, and about once a week, the therapeutic doses of about 20-25 IU/kg, about 25-30 IU/kg, about 30-35 IU/kg, about 35-40 IU/kg, 40-45 IU/kg, about 45-50 IU/kg, about 20 IU/kg, about 25 IU/kg, about 30 IU/kg, about 35 IU/kg, about 40 IU/kg, about 45 IU/kg, and about 50 IU/kg are envisioned.

Preferred doses and dosing intervals of the present invention are as follows:

about 10-25 IU/kg about once a day, about 10-25 IU/kg about 4 times a week, about 10-25 IU/kg about every 2 days, about 10-25 IU/kg about every 3 days, about 10-25 IU/kg about twice a week, about 10 IU/kg about once a day, about 10 IU/kg about 4 times a week, about 10 IU/kg about every 2 days, about 10 IU/kg about every 3 days, about 10 IU/kg about twice a week, about 20 IU/kg about once a day, about 20 IU/kg about 4 times a week, about 20 IU/kg about every 2 days, about 20 IU/kg about every 3 days, about 20 IU/kg about twice a week, about 20 IU/kg about every 5 days, about 20 IU/kg about once a week, about 25-50 IU/kg about once a day, about 25-50 IU/kg about 4 times a week, about 25-50 IU/kg about every 2 days, about 25-50 IU/kg about every 3 days, about 25-50 IU/kg about twice a week, about 25-50 IU/kg about every 5 days, about 25-50 IU/kg about once a week, about 25 IU/kg about once a day, about 25 IU/kg about 4 times a week, about 25 IU/kg about every 2 days, about 25 IU/kg about every 3 days, about 25 IU/kg about twice a week, about 25 IU/kg about every 5 days, about 25 IU/kg about once a week, about 50 IU/kg about once a day, about 50 IU/kg about 4 times a week, about 50 IU/kg about every 2 days, about 50 IU/kg about every 3 days, about 50 IU/kg about twice a week, about 50 IU/kg about every 5 days, and about 50 IU/kg about once a week.

"About once a week" includes about once every 6 to 8 days and about once every 7 days.

A dose of 25 IU/kg is the most preferred dose with a dosing interval of once a day, once every 2 days or once every 3 days.

As shown in Example 1, the preclinical bioavailability data for subcutaneous rIX-FP varied widely between animal models (e.g., 9.29% and 17% in monkeys for 500 IU/kg and 125 IU/kg, respectively, versus 43% and 55% in rabbits for 500 IU/kg and 150 IU/kg, respectively). The clinical data in Example 2 show, for the first time, which subcutaneous doses of long-acting FIX are effective in controlling hemophilia B in human patients.

Encompassed in this invention is an embodiment, wherein the median plasma level of FIX activity maintains a trough of at least about 5% above baseline for the entire dosing interval of once a day, 4 times a week, every 2 days or every 3 days, twice a week, after administration of a 10 IU/kg rIX-FP dose. In another embodiment, the median plasma level of FIX activity maintains a trough of at least about 5% above baseline for the dosing interval of once a day, 4 times a week, every 2 days or every 3 days, twice a week, every 5 days, or once a week after administration of a 25 IU/kg or 50 IU/kg rIX-FP dose.

Also encompassed in this invention is an embodiment, where a 10 IU/kg rIX-FP dose is applied about every day, a median plasma level of FIX activity of at least about 7% above baseline is maintained for the entire dosing interval. In another embodiment, where a 10 IU/kg rIX-FP dose is applied about four times a week, a median plasma level of FIX activity of at least about 4% above baseline is maintained for the entire dosing interval. In another embodiment, where a 10 IU/kg rIX-FP dose is applied about once every 2 days, the median plasma level of FIX activity maintains a trough of at least about 3% above baseline for the entire dosing interval. In a further embodiment, where a 10 IU/kg rIX-FP dose is applied about every 3 days, the median plasma level of FIX activity maintains a trough of at least about 2% above baseline for the entire dosing interval. In another embodiment, where a 10 IU/kg rIX-FP dose is applied about twice a week, a median plasma level of FIX activity of at least about 2% above baseline is maintained for the entire dosing interval. In a further embodiment, where a 10 IU/kg rIX-FP dose is applies about once every 5 days, the median plasma level of FIX activity maintains a trough of at least about 1.5%. In a further embodiment, where a 10 IU/kg rIX-FP dose is applied about once a week (e.g., every 7 days), the median plasma level of FIX activity maintains a trough of at least about 1% above baseline for the entire dosing interval.

In another embodiment, where a 25 IU/kg rIX-FP dose is applied about every day, the median plasma level of FIX activity maintains a trough of at least about 17% above baseline for the entire dosing interval. In a further embodiment, where a 25 IU/kg rIX-FP dose is applied about 4 times a week, the median plasma level of FIX activity maintains a trough of at least about 10% above baseline for the entire dosing interval. In a further embodiment, where a 25 IU/kg rIX-FP dose is applies about once every 2 days, the median plasma level of FIX activity maintains a trough of at least about 8%. In a further embodiment, where a 25 IU/kg rIX-FP dose is applies about once every 3 days, the median plasma level of FIX activity maintains a trough of at least about 5%. In a further embodiment, where a 25 IU/kg rIX-FP dose is applied about twice a week, the median plasma level of FIX activity maintains a trough of at least about 4 % above baseline for the entire dosing interval. In a further embodiment, where a 25 IU/kg rIX-FP dose is applies about once every 5 days, the median plasma level of FIX activity maintains a trough of at least about 3%. In a further embodiment, where a 25 IU/kg rIX-FP dose is applied about once a week (e.g., every 7 days), the median plasma level of FIX activity maintains a trough of at least about 2 % above baseline for the entire dosing interval.

In further embodiment, where a 50 IU/kg rIX-FP dose is applied about every day, the median plasma level of FIX activity maintains a trough of at least about 20% above baseline for the entire dosing interval. In another embodiment, where a 50 IU/kg rIX-FP dose is applied about 4 times a week, the median plasma level of FIX activity maintains a trough of at least about 12% above baseline for the entire dosing interval. In a further embodiment, where a 50 IU/kg rIX-FP dose is applies about once every 2 days, the median plasma level of FIX activity maintains a trough of at least about 10%. In a further embodiment, where a 50 IU/kg rIX-FP dose is applies about once every 3 days, the median plasma level of FIX activity maintains a trough of at least about 6%. In still a further embodiment, where a 50 IU/kg rIX-FP dose is applied about twice a week, the median plasma level of FIX activity maintains a trough of at least about 5% above baseline for the entire dosing interval. In a further embodiment, where a 50 IU/kg rIX-FP dose is applies about once every 5 days, the median plasma level of FIX activity maintains a trough of at least about 4%. In still a further embodiment, where a 50 IU/kg rIX-FP dose is applied about once a week (e.g., 7 days), the median plasma level of FIX activity maintains a trough of at least about 2% above baseline for the entire dosing interval.

Alternatively, in these embodiments, the plasma level of FIX activity could be calculated as a mean.

In particular, the present invention provides prophylactic dosing regimens for a fusion protein comprising human FIX and the human albumin, wherein the human Factor IX (FIX) portion is connected to the human albumin, wherein the human FIX is connected to the N-terminus of human albumin via a peptide linker cleavable by proteases involved in coagulation or activated by coagulation enzymes, wherein the fusion protein is to be administered subcutaneously to the subject at a dose of about 10-50 IU/kg using a dosing interval of once a day to about once every week.

rIX-FP fusion proteins with a linker which allows cleavage before activation of FIX, show an increased half-life of FIX due to albumin but since cleavage occurs before the bleeding event, the half-life of activated FIX is reduced before activation. If on the other hand, the cleavage occurs after activation of FIX, the albumin increases the half-life of FIX but the activated FIX is still fused to albumin, so it has low activity.

Proteolytic cleavage in a coagulation-related mode, in the sense of the invention, is any proteolytic cleavage that occurs as a consequence of the activation of at least one coagulation factor or coagulation cofactor. The coagulation factor is activated almost in parallel to the proteolytic cleavage of the linker peptide. Activation may occur, for example by proteolytic cleavage of the coagulation factor or by binding to a cofactor. The albumin increases the half-life of FIX in the blood until a bleeding event occurs, the bleeding event simultaneously activates FIX and cleaves it from albumin. The cleavage liberates the polypeptide from any activity-compromising steric hindrance caused by the albumin and thereby allows the generation of fusion proteins, which retain a high molar specific activity of the FIX. The cleaved FIX is then rapidly cleared from the blood due to the loss of albumin. Such fusion proteins exhibit improved half-life and molar specific activities that are increased in comparison to their non-cleavable counterparts. Thus, less rIX-FP is needed to provide a therapeutic effect compared to non-cleavable fusion proteins comprising rIX.

Preferred fusion proteins according to the invention are those that have a molar specific activity, in particular a molar specific coagulation-related activity of the therapeutic fusion protein that is increased at least 25% compared to that of the therapeutic fusion protein linked by a non-cleavable linker having the amino acid sequence GGGGGGV (SEQ ID NO: 4) in at least one coagulation-related assay. More preferred are fusion proteins in which the molar specific activity is increased by at least 50%, even more preferred those in which the molar specific activity is increased by at least 100%, in at least one of the different coagulation-related assays available.

In a further embodiment, the linker peptide comprises cleavage sites for more than one protease. This can be achieved either by a linker peptide that can be cleaved at the same position by different proteases or by a linker peptide that provides two or more different cleavage sites. There may be advantageous circumstances where the therapeutic fusion protein must be activated by proteolytic cleavage to achieve enzymatic activity and where different proteases may contribute to this activation step. Activation of FIX can either be achieved by FXIa or by FVIIa/Tissue Factor (TF). In a preferred embodiment, the linker is cleavable by FIXa and/or by FVIIa/Tissue Factor (TF).

rIX-FP (rFIX-albumin fusion protein) is described in WO2015/095925 as having the sequence set forth in SEQ ID NO: 3. It has prolonged circulation in plasma as shown by the 5.3-fold longer half-life ($t_{1/2}$), the 7-fold reduced CL, and the 7-fold greater AUC compared to rFIX (e.g., Benefix®). Moreover, when comparing pharmacokinetic parameters of rIX-FP to the corresponding pharmacokinetic data of FIX-Fc in the Alprolix® FDA prescribing information, rIX-FP has an about 3.8-fold higher AUC0-inf, an about 3.7-fold reduced CL/BW, an about 26% higher incremental recovery, an about 3.2-fold lower volume of distribution, and an increased mean residence time of the drug by about 19%, when compared to rIX-Fc (ALPROLIX®).

Prophylaxis is the treatment by injection of factor concentrate in order to prevent anticipated bleeding. Prophylaxis was conceived from the observation that moderate hemophilia patients with clotting factor level >1 IU/dl (>1%) seldom experience spontaneous bleeding and have much better preservation of joint function. Therefore, prophylaxis with FIX activity maintained above 1% to prevent bleeding and joint destruction should be the goal of therapy to preserve normal musculoskeletal function (GUIDELINES FOR THE MANAGEMENT OF HEMOPHILIA, 2nd edition, Prepared by the Treatment Guidelines Working Group, on behalf of the World Federation of Hemophilia (WFH)).

The present invention relates to clinically effective subcutaneous dosing regimens of fusion proteins comprising human FIX and human albumin, for use in a method of preventing bleeding in a human patient in a prophylactic dosing regimen. These findings further improve hemophilia prophylactic treatment since it allows the hemophilic patient to self-administer FIX, requiring no hospital or doctor visits.

In preferred embodiments, the plasma level of the FIX is maintained at a trough of at least about 0.5%, at least about 1%, at least about 2%, at least about 3%, at least about 4%, at least about 5%, at least about 7%, at least about 8%, at least about 10%, at least about 12%, at least about 15%, at least about 18%, at least about 20%, or at least about 30% above baseline for the entire dosing interval, preferably between 8 and 34%, or between 4 and 17 % or between 2.5 and 12 % above baseline for the entire dosing interval.

## Human FIX

Human FIX, one member of the group of vitamin K-dependent polypeptides, is a single-chain glycoprotein with a molecular weight of 57 kDa, which is secreted by liver cells into the blood stream as an inactive zymogen of 415 amino acids. It contains 12 $\gamma$-carboxy-glutamic acid residues localized in the N-terminal Gla-domain of the polypeptide. The Gla residues require vitamin K for their biosynthesis. Following the Gla domain there are two epidermal growth factor domains, an activation peptide, and a trypsin-type serine protease domain. Further posttranslational modifications of FIX encompass hydroxylation (Asp 64), N- (Asn157 and Asn167) as well as O-type glycosylation (Ser53, Ser61, Thr159, Thr169, and Thr172), sulfation (Tyr155), and phosphorylation (Ser158).

FIX is converted to its active form, Factor IXa, by proteolysis of the activation peptide at Arg145-Ala146 and Arg180-Val181 leading to the formation of two polypeptide chains, an N-terminal light chain (18 kDa) and a C-terminal heavy chain (28 kDa), which are held together by one disulfide bridge. Activation cleavage of Factor IX can be achieved in vitro e.g., by Factor XIa or Factor VIIa/TF. Factor IX is present in human plasma in a concentration of 5-10 $\mu$g/ml. Terminal plasma half-life of Factor IX in humans was found to be about 15 to 18 hours (White GC et al. 1997. Thromb Haemost. 78: 261-265; Ewenstein BM et al. 2002. Pharmacokinetic analysis of plasma-derived and recombinant F IX concentrates in previously treated patients with moderate or severe hemophilia B. Transfusion 42:190-197).

## Human albumin

Albumin, albumin family members and immunoglobulins and their fragments or derivatives have been described above as examples of half-life enhancing polypeptides (HLEPs). The terms "human serum albumin" (HSA) and "human albumin" (HA) are used interchangeably in this application.

As used herein, "albumin" refers collectively to albumin polypeptide or amino acid sequence, or an albumin fragment or variant having one or more functional activities (e.g., biological activities) of albumin. In particular, "albumin" refers to human albumin or fragments thereof, especially the mature form of human albumin. For example, albumin can have a sequence or variant thereof, as described in US2008260755A1, which is herein incorporated by reference in its entirety. The albumin portion of the albumin fusion proteins may comprise the full length of the HA sequence, or may include one or more fragments thereof that are capable of stabilizing or prolonging the therapeutic activity. Such fragments may be of 10 or more amino acids in length or may include about 15, 20, 25, 30, 50, or more contiguous amino acids from the HA sequence or may include part or all of specific domains of HA.

The albumin portion of the albumin fusion proteins of the invention may be a variant of normal HA, either natural or artificial. The therapeutic polypeptide portion of the fusion proteins of the invention may also be variants of the corresponding therapeutic polypeptides as described herein. The term "variants" includes insertions, deletions, and substitutions, either conservative or non-conservative, either natural or artificial, where such changes do not substantially alter the active site, or active domain that confers the therapeutic activities of the therapeutic polypeptides, as described in US2008260755A1, which is herein incorporated by reference in its entirety.

The invention specifically relates to fusion proteins comprising a) human Factor IX (FIX), and b) human albumin, wherein the human FIX is connected to the N-terminus of human albumin via a peptide linker cleavable by proteases involved in coagulation or activated by coagulation enzymes. The fusion protein has an increased in vivo half-life compared to the coagulation factor which has not been linked to albumin and the fusion protein has an at least 25% higher molar specific activity compared to the corresponding fusion protein with non-cleavable linker in at least one of the different coagulation-related assays available.

"Human Factor IX" within the above definition includes polypeptides that have the natural amino acid sequence including any natural polymorphisms. It also includes polypeptides with a slightly modified amino acid sequence, for instance, a modified N-terminal or C-terminal end including terminal amino acid deletions or additions, as long as those polypeptides substantially retain the activity of the respective therapeutic polypeptide. Variants included differ in one or more amino acid residues from the wild type sequence. Examples of such differences may include truncation of the N- and/or C-terminus by one or more amino acid residues (e.g. preferably 1 to 30 amino acid residues), or addition of one or more extra residues at the N- and/or C-terminus, as well as conservative amino acid substitutions, i.e., substitutions performed within groups of amino acids with similar characteristics, e.g. (1) small amino acids, (2) acidic amino acids, (3) polar amino acids, (4) basic amino acids, (5) hydrophobic amino acids, and (6) aromatic amino acids. Examples of such conservative substitutions are shown in the following table.

**Table 1: Conservative substitutions of amino acids**

| (1) | Alanine | Glycine | | |
|-----|---------|---------|---|---|
| (2) | Aspartic acid | Glutamic acid | | |
| (3a) | Asparagine | Glutamine | | |
| (3b) | Serine | Threonine | | |
| (4) | Arginine | Histidine | Lysine | |
| (5) | Isoleucine | Leucine | Methionine | Valine |
| (6) | Phenylalanine | Tyrosine | Tryptophan | |

The in vivo half-life of the fusion proteins of the invention, in general determined as terminal half-life or β-half-life, is usually at least about 25%, preferably at least about 50%, and more preferably more than 100% higher than the in vivo half-life of the non-fused polypeptide.

The fusion proteins of the present invention have at least a 25%, preferably at least a 50%, more preferably an at least 100% increased molar specific activity compared to the corresponding fusion proteins without cleavable linkers.

The molar specific activity (or molar specific coagulation-related activity as considered here in particular) in this regard is defined as the activity expressed per mole (or e.g. nmole) of the therapeutic polypeptide or therapeutic fusion protein of interest. Calculation of the molar specific activity allows a direct comparison of the activity of the different constructs which are not affected by the different molecular weights or optical densities of the polypeptides studied. The molar specific activity may be calculated as exemplified in Table 2 below for FIX and a rIX-FP fusion protein.

**Table 2: Calculation of molar specific activity as shown for a purified FIX-HSA fusion protein**

| Product | $OD_{(280nm, 1\%)}$ | MW | Activity/Vol/$OD_{280}$ (IU/L/$OD_{280}$) | Molar optical density ($OD_{(280)}$ at 1 mol/L) | Calculation of molar specific activity (IU/mol) |
|---------|------|-----|----------------|------------------|-------------------|
| FIX | 13.3 [1] | 57 000 | determined for product | 75810 (= MW x $OD_{(280, 1\%)}$/10) | =(Activity/Vol/$OD_{280}$) x ($OD_{280}$ at 1 mol/L) |
| Albumin | 5.7 [2] | 66 300 | | 37791 (= MW x $OD_{(280, 1\%)}$/10) | |
| FIX-FP | | | determined for product | 113601 (= sum of molar optical density of FIX and albumin) | = (Activity/Vol/$OD_{280}$) x ($OD_{280}$ at 1 mol/L) |
| 1) R.G. Di Scipio et al., Biochem. 16:6698-706 (1977) | | | | | |
| 2) C. Chaudhury et al. J. Exp. Med. 197(3):315-322 (2003) | | | | | |

In order to determine a molar specific coagulation-related activity, any assay may be used that determines enzymatic or cofactor activities that are relevant to the coagulation process.

Therefore "coagulation-related assays" in the sense of the invention is any assay which determines enzymatic or cofactor activities that are of relevance in the coagulation process or that is able to determine that either the intrinsic or the extrinsic coagulation cascade has been activated. The "coagulation-related" assay thus may be direct coagulation assays like aPTT, PT, or the thrombin generation assays. However, other assays like, e.g., chromogenic assays applied for specific coagulation factors are also included. Examples for such assays or corresponding reagents are Pathromtin® SL (aPTT assay, Dade Behring) or Thromborel® S (Prothrombin time assay, Dade Behring) with corresponding coagulation factor deficient plasma (Dade Behring), Thrombin generation assay kits (Technoclone, Thrombinoscope) using e.g. coagulation factor deficient plasma, chromogenic assays like Biophen Factor IX (Hyphen BioMed), Staclot® FVIIa-rTF (Roche Diagnostics GmbH), Coatest® Factor VIII:C/4 (Chromogenix), or others.

For purposes of this invention, an increase in any one of the above assays or an equivalent coagulation-related assay is considered to show an increase in molar specific activity. For example, a 25% increase refers to a 25% increase in any of the above or an equivalent assay.

To determine whether therapeutic fusion proteins fall within the scope of the present invention, the standard against which the molar specific activity of these proteins is compared is a construct in which the respective coagulation factor and the human albumin is linked by a non-cleavable linker having the amino acid sequence GGGGGGV (SEQ ID NO: 4).

For FIX, aPTT assays are often used for determination of coagulation activity. However, other coagulation-related assays or assay principles may be applied to determine molar specific activity for FIX.

Although it is desirable to have a high in vivo recovery and a long half-life for a non-activated coagulation factor, it is advantageous to limit the half-life of a coagulation factor after its activation or the activation of its co-factor in order to avoid a prothrombotic risk. Therefore, after the coagulation process has been initiated, the half-life of the active coagulation factor should again be reduced. This can either be achieved by enhancing inactivation in a coagulation-related mode or by elimination of the coagulation factor.

Inactivation according to the present invention means the decrease of activity of the therapeutic polypeptide which can be caused, for example, by a complex formation of a coagulation factor and an inhibitor of the corresponding coagulation factor or by further proteolytic cleavage as known, e.g., in the case of FVIII and FV.

The inactivation rate of an activated therapeutic fusion protein is defined as the rate the activity is declining, e.g., by reaction with inhibitors or by proteolytic inactivation. The inactivation rate may be measured by following the molar specific activity of the activated coagulation factor over time in the presence of physiologic amounts of inhibitors of this coagulation factor.

Alternatively, the inactivation rate may be determined after administration of the activated product to an animal followed by testing of plasma samples at an appropriate time frame using activity and antigen assays.

When for therapeutic fusion proteins a determination is needed whether these proteins fall within the scope of the present invention, the standard against which the inactivation rate of these therapeutic proteins is compared to, is a construct in which the respective coagulation factor and human albumin is joined by a non-cleavable linker having the amino acid sequence GGGGGGV (SEQ ID NO: 4).

The elimination rate of an activated therapeutic fusion protein is defined as the rate the polypeptide is eliminated from the circulation of humans or animals. The elimination rate may be determined by measuring the pharmacokinetics of the activated, therapeutic fusion protein after subcutaneous administration. Using an antigen assay, the elimination by direct removal from the circulation can be determined. An activity assay may be additionally used to determine the inactivation rate.

When for therapeutic fusion proteins a determination is needed whether these proteins fall within the scope of the present invention, the standard against which the elimination rate of these proteins is compared to, is a construct in which the respective coagulation factor and the human albumin is joined by the non-cleavable linker having the amino acid sequence GGGGGGV (SEQ ID NO: 4).

According to this invention, the fusions protein comprises human FIX connected to the N-terminus of human albumin via a peptide linker cleavable by proteases involved in coagulation or activated by coagulation enzymes. The linker should be non-immunogenic and should be flexible enough to allow cleavage by proteases.

The cleavable linker preferably comprises a sequence derived from a) the therapeutic polypeptide to be administered itself if it contains proteolytic cleavage sites that are proteolytically cleaved during activation of the therapeutic polypeptide, b) a substrate polypeptide of this therapeutic polypeptide, or c) a substrate polypeptide cleaved by a protease which is activated or formed by the direct or indirect involvement of the therapeutic polypeptide.

The linker region in a more preferred embodiment comprises a sequence of the therapeutic polypeptide to be applied, which should result in a decreased risk of neoantigenic properties of the expressed fusion protein.

In a preferred embodiment, the linker sequence is either derived from the sequences of the activation regions of FIX, from the cleavage region of any substrate of FIX like FX or FVII or from the cleavage region of any substrate polypeptide that is cleaved by a protease in whose activation FIXa is involved.

In a highly preferred embodiment the linker peptide is derived from FIX itself. In a further preferred embodiment the linker peptide is derived from FX or FVII. In still a further preferred embodiment the linker sequence comprises two cleavage sequences that can be cleaved by FXIa or FVIIa/TF, two physiologically relevant activators of FIX.

Variants and fragments of the described linkers are also encompassed in the present invention as long as the linker can still be cleaved by the protease or the proteases that cleave the linkers. The term "variants" includes insertions, deletions and substitutions, either conservative or non-conservative.

## Pharmaceutical Compositions and Modes of Administration

The fusion proteins of the invention can be incorporated into pharmaceutical compositions suitable for subcutaneous administration. Such compositions typically comprise the protein and a pharmaceutically acceptable carrier. As used herein, "pharmaceutically acceptable carrier" is intended to include any and all solvents, dispersion media, coatings, antibacterial and antifungal agents, isotonic and absorption delaying agents, and the like, compatible with pharmaceutical subcutaneous administration. Solutions or suspensions used for subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid (EDTA); buffers such as acetates, citrates or phosphates, and agents for the adjustment of tonicity such as sodium chloride or dextrose. Suitable carriers are described in the most recent edition of Remington's Pharmaceutical Sciences, a standard reference text in the field, which is incorporated herein by reference. Preferred examples of such carriers or diluents include, but are not limited to, water, saline, Ringer's solutions, dextrose solution, and 5% human serum albumin. Liposomes and non-aqueous vehicles such as fixed oils may also be used. The use of such media and agents for pharmaceutically active substances is well known in the art. Except insofar as any conventional media or agent is incompatible with the active compound, use thereof in the compositions is contemplated. Supplementary active compounds can also be incorporated into the compositions. The pH can be adjusted with acids or bases; such as hydrochloric acid of sodium hydroxide. The parenteral preparation can be enclosed in ampoules, disposable syringes or multiple dose vials made of glass of plastic. Administration as a subcutaneous injection is the preferred route of administration.

Pharmaceutical compositions suitable for injectable use include sterile aqueous solutions (where water soluble) or dispersions and sterile powders for the extemporaneous preparation of sterile injectable solutions or dispersion. For subcutaneous administration, suitable carriers include physiological saline, bacteriostatic water, Cremophor EL (BASF, Parsippany, N.J.) or phosphate buffered saline (PBS). In all cases, the composition must be sterile and should be fluid to the extent that easy syringe ability exists. It must be stable under the conditions of manufacture and storage and must be preserved against the contaminating action of microorganisms such as bacteria and fungi. The carrier can be a solvent or dispersion medium containing, for examples, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound (e.g., rIX-FP) in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, methods of preparation are vacuum drying and freeze-drying that yields a powder of the active ingredient plus any additional desired ingredient from a previously sterile-filtered solution thereof.

The formulations for injection can contain the Factor IX (FIX) fusion protein (e.g., rIX-FP) in a therapeutically effective amount, which amount may be determined by the skilled person. In particular, the Factor IX (FIX) fusion protein (i.e., rIX-FP) may administered at a concentration of about 100 to 2000 IU/ml. For example, the fusion protein may be provided for administration at a concentration of about 100 to 400 IU/ml, or about 100, 200 or 400 IU/ml. The fusion protein may also be provided for administration at about 600 IU/ml, 800 IU/ml, 1000 IU/ml, 1200 IU/ml, 1400 IU/ml, 1600 IU/ml, 1800 IU/ml or 2000 IU/ml. The fusion protein may also be provided for administration at about 2500 IU/ml, 3000 IU/ml, 3500 IU/ml or 4000 IU/ml.

It is especially advantageous to formulate pharmaceutical compositions, such as compositions for injection, in dosage unit form for ease of administration and uniformity of dosage. Dosage unit form as used herein refers to physically discrete units suited as unitary dosages for the patient to be treated; each unit containing a predetermined quantity of active compound calculated to produce the desired therapeutic effect in association with the required pharmaceutical carrier. The specification for the dosage unit forms are dictated by and directly dependent on the unique characteristics of the active compound and the particular therapeutic effect to be achieved.

The pharmaceutical compositions can be included in a container, pack, or dispenser together with instructions for administration.

The invention will be more clearly understood by reference to the following examples, which are included herewith for purposes of illustration only and are not intended to be limiting of the invention. All patents and publications referred to herein are expressly incorporated by reference.

**Examples**

**Example 1 - Preclinical Studies**

**A. Pharmacokinetics of rIX-FP and Berinin® P following a single subcutaneous or intravenous injection to rabbits**

The aim of this study was to investigate the bioavailability of rIX-FP following subcutaneous administration to rabbits and furthermore compare the subcutaneous pharmacokinetic profile with a marketed plasma-derived human coagulation factor IX (FIX) product (i.e. Berinin® P).

Four groups of rabbits, each comprising of 3 female animals, received either a single subcutaneous (s.c.) dose at a dose level of 150 IU/kg (rIX-FP) and 500 IU/kg (rIX-FP and Berinin® P), respectively, or a single intravenous (i.v.) dose at a dose level of 150 IU/kg (rIX-FP). Thereafter, blood samples were drawn up to 10 days. The subsequent determination of human FIX antigen concentrations in the rabbit plasma samples was performed using an enzyme-linked immunosorbent assay (ELISA) technique.

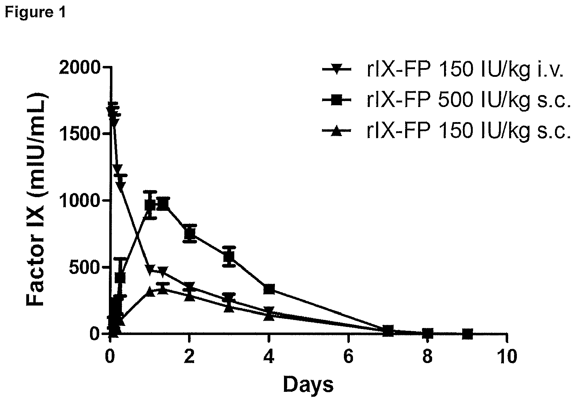

The results from this study showed that in rabbits, rIX-FP was transported to the vascular system following a subcutaneous injection. After s.c. administration of 150 and 500 IU/kg rIX-FP a dose-dependent increase in human FIX plasma levels was observed, reaching peak plasma levels between 24 and 32 hours post dosing (Figure 1). Thereby, the respective bioavailability of rIX-FP after s.c. application was 55% for the 150 IU/kg and 43% for the 500 IU/kg group (48% = geometric mean value). In comparison to a s.c. application of Berinin® P, human FIX plasma levels after s.c. administration of 500 IU/kg rIX-FP were higher and decreased less rapidly. Thereby, maximum human FIX plasma levels after s.c. rIX-FP dosing (1.01 IU/mL) were about six times higher than after Berinin® P dosing (0.17 IU/mL), which already reached peak plasma levels after -10 hours. Furthermore, the AUC after s.c. administration of rIX-FP was about 13 times higher compared to that of Berinin® P (Table 3). No adverse effects of treatment were observed following i.v. or s.c. administration of either rIX-FP or Berinin® P.

**Table 3: Geometric means of pharmacokinetic parameter estimates in rabbits**

| Group | $C_{max}$ [IU/mL] | $T_{max}$ [h] | $T_{½}$ [h] | $AUC_{0\text{-infin.}}$ [h*IU/mL] | $AUC_{first\text{-}last>0}$ [h*IU/mL] | Clearan ce [mL/h/kg] | F [%] |
|---|---|---|---|---|---|---|---|
| 1 Berinin®P, 500 IU/kg s.c. | 0.17 | 9.52 | 20 | 6 | 5 | 87.96 | N.a. |
| 2 rIX-FP, 500 IU/kg s.c. | 1.01 | 26.42 | 20 | 70 | 70 | 7.14 | 43 |
| 3 rIX-FP, 150 IU/kg s.c. | 0.34 | 29.07 | 29 | 27 | 26 | 5.59 | 55 |
| 4 rIX-FP, 150 IU/kg i.v. | 1.69 | 0.44 | 25 | 49 | 48 | 3.05 | N.a. |
| Parameters were estimated by non-compartmental methods, F= Bioavailability | | | | | | | |

Taken together, this study demonstrated a bioavailability of 43-55% after subcutaneous administration of rIX-FP to rabbits. S.c. administration of rIX-FP was well tolerated, had a pharmacologically-relevant bioavailability and overall exposure was superior to the marketed plasma-derived FIX product, Berinin® P.

**B. Pharmacokinetics of CSL654 (rIX-FP) after i.v. and s.c. administration to pigs**

The aim of this study was to investigate the pharmacokinetic characteristics (including subcutaneous bioavailability) of rIX-FP following intravenous and subcutaneous administration to pigs.

Two groups of pigs, each comprised of 3 male animals, received either a single intravenous (i.v.) dose of 250 IU/kg rIX-FP or a single subcutaneous (s.c.) dose of 241.5 IU/kg rIX-FP. Thereafter, coagulation factor IX (FIX) plasma levels were followed up for 17 days post dosing based on determination of FIX antigen concentrations using an enzyme-linked immunosorbant assay (ELISA) method.

rIX-FP administration to pigs was well tolerated, i.e. no adverse effects of treatment were observed following i.v. or s.c. administration.

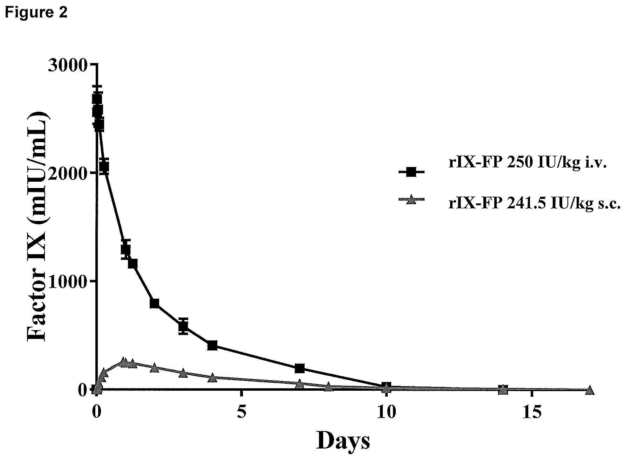

After s.c. administration of rIX-FP, peak FIX plasma levels were reached after around 22 hours (Figure 2, Table 4). The respective bioavailability of rIX-FP after s.c. application was 22%. Terminal half-lives were comparable after s.c. (53h) and i.v. (44h) dosing.

**Table 4: Geometric means of pharmacokinetic parameter estimates in pigs**

| Group | $T_{max}$ [h] | $C_{max}$ [IU/mL] | $AUC_{0\text{-infin.}}$ [h*mg/mL] | Clearance [mL/h/kg] | $T_{1/2}$ [h] | F [%] |
|---|---|---|---|---|---|---|
| **1** rIX-FP, 250 IU/kg i.v. | 0.08 | 2.68 | 124 | 2.0 | 44 | - |
| **2** rIX-FP, 241.5 IU/kg s.c. | 22 | 0.26 | 26 | 9.1 | 53 | 22 |
| Parameters were estimated by non-compartmental methods. | | | | | | |

Pharmacokinetic investigations revealed a bioavailability of 22% after s.c. administration of rIX-FP whilst terminal half-life was comparable to an i.v. dosing.

### C. Pharmacokinetics of rIX-FP/BeneFIX® after i.v. and s.c. administration to primates

This study was designed to assess the pharmacokinetics of rIX-FP following single intravenous and subcutaneous doses to male and female Cynomolgus monkeys. BeneFIX® is a marketed product and was used as a reference test item.

Seven groups of cynomolgus monkeys, each comprising of one male and one female animal, received single intravenous or subcutaneous doses of rIX-FP or BeneFIX® at dose levels in the range of 125 IU/kg and 500 IU/kg. Blood samples were taken from each monkey over a 10 day period. Due to incorrect preparation of formulations in Groups 1, 2 and 4, only plasma samples of Groups 3, 5, 6 and 7 were analyzed.

Determination of human factor IX concentrations in the monkey plasma samples were performed using validated ELISA methods.

No local intolerabilities were observed following administration of either rIX-FP or BeneFIX®.

The pharmacokinetic concentration data following intravenous administration of rIX-FP and subcutaneous administration of rIX-FP and BeneFIX® were variable and it was difficult to ascertain when a return to background levels were achieved due to the level of endogenous factor IX detected in the samples and the high level of variability of endogenous factor IX levels between individual animals. Therefore, these data were not amenable to full pharmacokinetic data analysis. To account for endogenous level of factor IX, plasma concentrations of factor IX for each monkey were corrected by subtracting the concentrations at pre-dose from the measured concentration at each time point. These baseline-corrected values were used to calculate the Cmax and AUCt values of rIX-FP and BeneFIX®, and the bioavailability of rIX-FP.

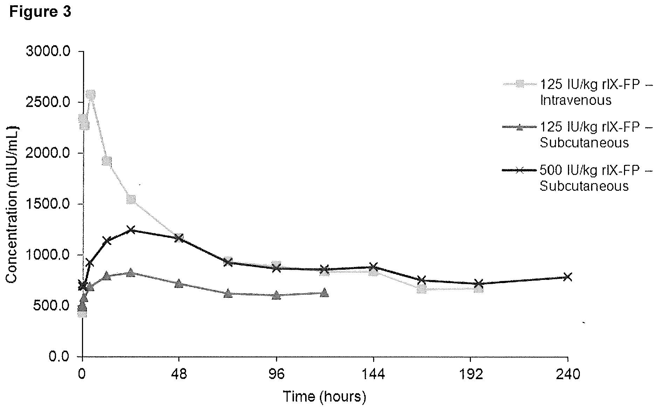

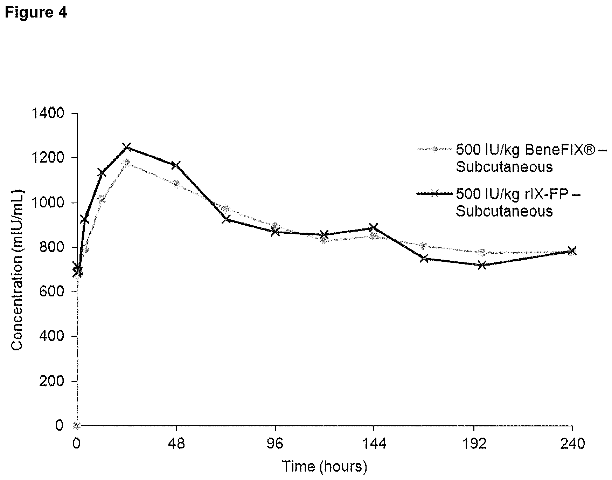

Mean plasma concentration-time curves of rIX-FP and BeneFIX® after intravenous and subcutaneous administration are presented in Figures 3 and 4. The pharmacokinetic parameters derived from these data are presented in Table 5.

**Table 5: The mean pharmacokinetic parameters in cynomolgus monkeys**

| Group | Test substance | Dose route | Dose level (IU/kg) | $C_{max}$ (mIU/mL) | $AUC_t$ (mIU.h/mL) | F % |
|---|---|---|---|---|---|---|
| 3 | BeneFIX® | Subcutaneous | 500 | 501 | 37300 | - |
| 5 | rIX-FP | Intravenous | 125 | 2420 | 118000 | - |
| 6 | rIX-FP | Subcutaneous | 125 | 317 | 19700 | 17.0 |
| 7 | rIX-FP | Subcutaneous | 500 | 534 | 44600 | 9.29 |

Taken together, it can be concluded that the application of the study drugs was well tolerated, that the Cmax and AUCt values following subcutaneous administration of rIX FP were higher than the respective values after administration of BeneFIX®, and that following subcutaneous administration of rIX-FP, the bioavailability was 17.0% and 9.29% at dose levels of 125 IU/kg and 500 IU/kg, respectively (mean value: 13%).

### D. Bioavailability of rIX-FP after subcutaneous administration to hemophilia B mice

The aim of this study was to investigate the bioavailability of rIX-FP following subcutaneous administration to hemophilia B mice and furthermore compare its subcutaneous pharmacokinetic profile with that of a marketed recombinant human coagulation factor IX (FIX) product (i.e. BeneFIX®).

Five groups of hemophilia B mice, each comprising 12-15 female or male animals, received either a single subcutaneous (s.c.) dose at a dose level of 125 IU/kg (rIX-FP) and 250 IU/kg (rIX-FP and BeneFIX®), respectively, or a single intravenous (i.v.) dose of 125 IU/kg (rIX-FP). Thereafter, blood samples were drawn for up to four days. The subsequent determination of human FIX activity and antigen concentrations in the murine plasma samples was performed using a clotting assay and an enzyme-linked immunosorbent assay (ELISA) technique, respectively.

In one part of this study, s.c. administration of rIX-FP was compared to the respective i.v. dosing. In the second part of the study, two different s.c. doses of rIX-FP were compared, whilst the highest dose was additionally compared to an equal dose of BeneFIX®.

No local or systemic adverse effects of treatment were observed following i.v. or s.c. administration of either rIX-FP or BeneFIX® during the study period.

The results from this study showed that in hemophilia B mice rIX-FP was transported to the vascular system following a subcutaneous injection.

Study part 1: After s.c. administration of 125 IU/kg rIX-FP, peak plasma levels were reached between 16 (activity levels) and 24 (antigen levels) hours post dosing (Tables 6 and 7). Respective bioavailability was 50 % for FIX activity levels and 31 % for FIX antigen levels.

Observed differences in PK values derived from activity and antigen FIX levels might be due to differences in assay sensitivity which was higher for the FIX activity assay in comparison to antigen measurements using ELISA.

Study part 2: After s.c. administration of 125 and 250 IU/kg rIX-FP, pharmacokinetics were dose-proportional using activity or antigen FIX plasma levels for estimations (Tables 6 and 7). Peak plasma levels were again reached between 16 and 24 hours.

In comparison to a s.c. application of BeneFIX®, human FIX activity plasma levels after s.c. administration of 250 IU/kg rIX-FP were higher and decreased less rapidly (Table 6). Thereby, maximum plasma levels of rIX-FP (0.69 IU/mL) were about seven times higher than for BeneFIX® (0.10 IU/mL). Furthermore, the AUC after s.c. administration of rIX-FP (38.6 h*IU/mL) was about eight times higher compared to BeneFIX® (4.6 h*IU/mL). Based on FIX antigen levels (Table 7), maximum FIX plasma levels after rIX-FP dosing (0.54 IU/mL) were about five times higher than after BeneFIX® (0.11 IU/mL), whilst the AUC after s.c. administration of rIX-FP (26.0 h*IU/mL) was about 15 times higher compared to BeneFIX® (1.7 h*IU/mL).

**Table 6: Estimates of pharmacokinetic parameters (FIX activity) in hemophilia B mice**

| Group | $C_{max}$ [IU/mL] | $T_{max}$ [h] | $T_{1/2}$ [h] | $AUC_{first - last>0}$ [h*IU/mL] | $AUC_{0-infin.}$ [h*IU/mL] | Clearance [mL/h/kg] | F [%] |
|---|---|---|---|---|---|---|---|
| 1. rIX-FP, 125 IU/kg i.v. | 1.77 | 0.08 | 20 | 34.4 | 36.0 | 3.48 | N.a. |
| 2. rIX-FP, 125 IU/kg s.c. | 0.36 | 16.0 | 30 | 16.0 | 18.1 | 6.91 | 50 |
| 3. rIX-FP, 125 IU/kg s.c. | 0.38 | 16.0 | N.c. | 21.6 | N.c. | N.c. | N.a. |
| 4. rIX-FP, 250 IU/kg s.c. | 0.69 | 24.0 | N.c. | 38.6 | N.c. | N.c. | N.a. |
| 5. BeneFIX®, 250 IU/kg s.c. | 0.10 | 32.0 | N.c. | 4.6 | N.c. | N.c. | N.a. |
| Parameters were estimated by non-compartmental methods<br>N.c.= not calculated | | | | | | | |

**Table 7: Estimates of pharmacokinetic parameters (human FIX antigen) in hemophilia B mice**

| Group | $C_{max}$ [IU/mL] | Tmax [h] | $T_{1/2}$ [h] | $AUC_{first - last>0}$ [h*IU/mL] | $AUC_{0-infin.}$ [h*IU/mL] | Clearance [mL/h/kg] | F [%] |
|---|---|---|---|---|---|---|---|
| 1. rIX-FP, 125 IU/kg i.v. | 1.89 | 0.08 | 20 | 23.1 | 24.1 | 5.2 | N.a. |
| 2. rIX-FP, 125 IU/kg s.c. | 0.18 | 24.0 | 28 | 6.6 | 7.4 | 16.9 | 31 |
| 3. rIX-FP, 125 IU/kg s.c. | 0.23 | 16.0 | 18 | 11.7 | 12.4 | 10.1 | N.a. |

(continued)

| Group | $C_{max}$ [IU/mL] | Tmax [h] | $T_{½}$ [h] | $AUC_{first - last>0}$ [h*IU/mL] | $AUC_{0-infin.}$ [h*IU/mL] | Clearance [mL/h/kg] | F [%] |
|---|---|---|---|---|---|---|---|
| 4. rIX-FP, 250 IU/kg s.c. | 0.54 | 16.0 | 32 | 26.0 | 30.3 | 8.2 | N.a. |
| 5. BeneFIX®, 250 IU/kg s.c. | 0.11 | 8.0 | 10 | 1.7 | 2.0 | 127.1 | N.a. |
| Parameters were estimated by non-compartmental methods | | | | | | | |

Taken together, s.c. administration of rIX-FP was well tolerated, had a pharmacologically-relevant bioavailability and pharmacokinetics were superior to the marketed recombinant FIX product BeneFIX®

**Example 2 - Clinical Studies**

***Bioavailability and Dosing Simulations of rIX-FP after subcutaneous administration to hemophilic B patients***

A Phase I pharmacokinetic study of subcutaneous administration of rIX-FP was conducted as an open-label, single ascending dose study to assess the pharmacokinetics and safety of rIX-FP following subcutaneous (s.c.) administration of 25 or 50 IU/kg rIX-FP in hemophilia B subjects (Cohorts 1 and 2, respectively). Repeat dose administration of 25 IU/kg given every 3 days for 15 doses was also assessed (Cohort 3).

The main eligibility criteria for subjects in the subcutaneous substudy were that the subjects were male, at least 18 years of age with body weight between 50.0 and 100.0 kg who are currently enrolled in Study CSL654_3003 with hemophilia B (FIX activity $\leq$ 2%), and have received rIX-FP for $\geq$ 100 EDs. Subjects who have experienced a life-threatening bleeding episode or had major surgery during the 3 months were ineligible for the study.

Subjects enrolled in the substudy were sequentially assigned to a cohort (Cohort 1 [single 25 IU/kg dose], Cohort 2 [single 50 IU/kg dose], or Cohort 3 [repeat dose administration of 25 IU/kg]).

**Single Dose Analyses (25 IU/kg and 50 IU/kg)**

For Cohorts 1 and 2, a single dose of rIX-FP was administered at least 14 days after the previous rIX-FP administration and blood samples for PK analysis were collected at predose, 30 minutes, 24, 48, 72, 120, 168, 240 and 336 hours after administration. Following collection of the last SC PK sample at the 336 hr time point on Day 15, a single IV dose of 50 IU/kg rIX-FP was administered and blood samples for PK analysis were collected at selected time points. PK was calculated using FIX activity, measured by a one-stage aPTT assay in the central lab (CSL Behring) and Pathromtin SL was used as the activator.

Subjects returned in 2 weeks (Day 28) for an end of substudy visit to assess safety. After the end of the substudy visit on Day 28, subjects returned to their IV rIX-FP prophylaxis regimen in the main study.

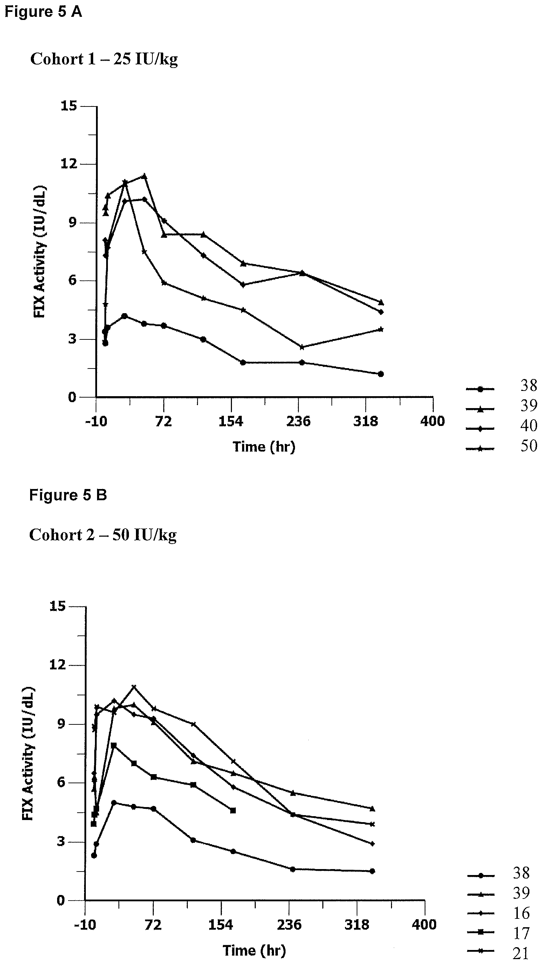

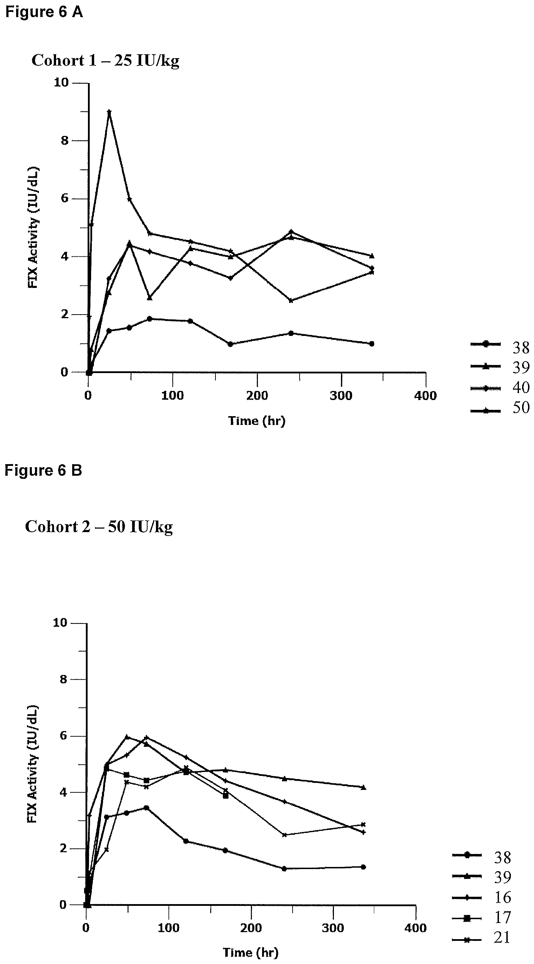

The pharmacokinetics of rIX-FP after subcutaneous administration was assessed on the basis of measurements of FIX activity level in plasma. Figure 5A and 5B shows uncorrected FIX Activity vs Time plot at (25 and 50 IU/kg) subcutaneous dose of rIX-FP in each human patient. The FIX activity levels were corrected for the exponential decline of the pre-dose FIX levels, especially since the baseline was higher than anticipated endogenous levels due to the fact that the patients came from a prior intravenous dosing study. Once corrected, x negative values (4 out of a total of 30 PK sample points) were set to a 0.01% threshold to enable model fitting. Figure 6A and 6B shows the corrected FIX Activity vs Time plot by ID at 25 and 50 IU/kg subcutaneous dose of rIX-FP in human patients. These graphs show high predose levels resulted from IV carryover prior to SC administration and that plasma level increases ranged from 2 to 8% above predose levels, across all dose levels, peaking 24-48 hours after the SC dose.

The corrected PK concentrations were used to analyze the following pharmacokinetic parameters, including Area under the concentration versus time curve (AUC), and Bioavailability using Non-compartmental Analysis (NCA) method in Phoenix WinNonlin software. The bioavailability was calculated using the dose normalized partial AUCs from 0-336 hr for both s.c. and IV dosing and it was between 14.12% and 102% for cohort 1 and 10.1 to 35.5 % for cohort 2% (see Table 8A) over baseline.

## Table 8A: Bioavailability

### Cohort1 (single 25 IU/kg)

| ID | AUCSC0-336 hr*IU/dL | SC Dose | AUCIV0-336 hr*IU/dL | IV Dose | F% |
|---|---|---|---|---|---|
| 38 | 445.83 | 25 | 3157.07 | 25 | 14.12 |
| 39 | 1305.41 | 25 | 3873.82 | 25 | 33.70 |
| 40 | 1283.13 | 25 | 3697.45 | 25 | 34.70 |
| 50* | 1425.30 | 25 | 2790.42 | 50 | 102.16 |

Avg – 27.4AUCSC0-336: Corrected partial Area under the curve (AUC) for the subcutaneous dosing from 0 to 336hrs (CSL654_3003 study)

AUCIV0-336: Corrected partial Area under the curve (AUC) for the IV dosing from 0 to 336hrs (CSL654_3001 study)

*Received 50IU/kg IV dose, Bioavailability was calculated with dose normalizing AUC

### Cohort 2 (single 50 IU/kg)

| ID | AUCSC0-336 hr*IU/dL | SC Dose | AUCIV0-336 hr*IU/dL | IV Dose | F% |
|---|---|---|---|---|---|
| 38 | 682.31 | 50 | 3157.07 | 25 | 10.80 |
| 39 | 1544.96 | 50 | 3873.82 | 25 | 19.94 |
| 16 | 1442.93 | 50 | 2952.75 | 25 | 24.43 |
| 17 | 1325.26 | 50 | 1866.07 | 25 | 35.51 |
| 21 | 1142.41 | 50 | 6726.73 | 50 | 16.98 |

Avg – 27.4AUCSC0-336: Corrected partial Area under the curve (AUC) for the subcutaneous dosing from 0 to 336hrs (CSL654_3003 study)

AUCIV0-336: Corrected partial Area under the curve (AUC) for the IV dosing from 0 to 336hrs (CSL654_3001 study)

**Pharmacokinetic Analyses**

Modeling and simulations were performed using the Phoenix WinNonlin software. For data presentation and construction of plots, Excel and R software were used.

The compartmental pharmacokinetic analysis was performed for each individual subject utilizing a naïve pooled approach. The initial estimates were obtained from NCA. Volume of distribution (V), clearance (CL) and Absorption rate constant (Ka) were estimated using compartmental model fitting.

Stepwise model selection was based on minimization of the objective function value (OFV). Model adequacy and performance were checked by various diagnostic plots such as observed (DV), individual predicted (IPRED) vs Time.

The final model was a one compartment additive error model which was further utilized for simulations. The parameter estimates for each subject are provided in Table 8B.

Due to limited sampling window up to 336 hr, an additional 4 time points were added after 336hr to estimate elimination assuming an exponential decline in the FIX activity levels similar to IV. With this assumption, the actual SC fraction absorbed may be greater than that estimated in the current model fits/simulations.

**Table 8B: Parameter estimates from the one compartment additive model**

| Cohort 1 (single 25 IU/kg) | | | | |
|---|---|---|---|---|
| Parameters | **38** | **39** | **40** | **50** |
| Clearance(dL/hr) | 2.11 | 1.08 | 0.77 | 0.89 |
| Volume (dL) | 56.09 | 140.06 | 24.04 | 2.53 |
| Ka (1/hr) | 0.0037 | 0.0057 | 0.0025 | 0.0046 |
| Cohort 2 (single 50 IU/kg) | | | | |
| Parameters | **38** | **39** | **16** | **21** |
| Clearance(dL/hr) | 3.00 | 1.76 | 1.92 | 1.96 |
| Volume (dL) | 45.95 | 37.71 | 9.98 | 97.85 |
| Ka (1/hr) | 0.0047 | 0.0029 | 0.0032 | 0.0042 |

**Repeat Dose Analyses (25 IU/kg every 3 days)**

For Cohort 3 the Dose 1of 25 IU/kg rIX-FP was administered at least 14 days after the previous rIX-FP administration and at 24 hours, the blood samples were analyzed for plasma FIX activity and activation of coagulation at predose, 30 minutes, 3 hours, 8 hours, 24 hours, 48 hours and 72 hours after administration.

Subjects in Cohort 3 returned to the study site for SC Doses 2 and 3 of 25IU/kg rIX-FP. Prior to SC Dose 3, a blood samples were analyzed for plasma FIX activity. Subjects administered SC doses 4 to 12 at home every 3 days and returned to the study site for SC Dose 13. Prior to SC Dose 13, blood samples for plasma FIX activity were also collected. After each dose administered in at the study site, the investigator assessed local tolerability (at 30 min) and recorded AEs and any concomitant therapies. Dose 14 was also administered at home.

SC Dose 15 of 25 IU/kg rIX-FP was given at the study site and blood samples were assessed for plasma FIX activity and activation of coagulation tests at pre-dose, 30 mins, 3 hours, 8 hours, 24 hours, 48 hours and 72 hours after administration. Assessment of local tolerability as well as AEs and any concomitant therapies were assessed at for each dose given at the study site.

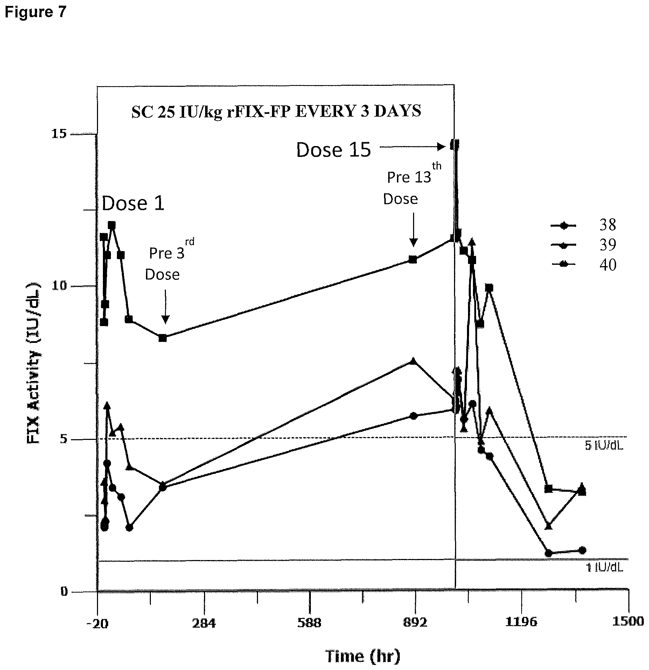

The FIX activity at each time point for each patient is shown in Table 9 and in Figure 7.

**Table 9: Observed FIX activity in patients in Cohort 3 administered 25 IU/kg every 3 days**

| Dose | 1 | 1 | 1 | 1 | 1 | 1 | 1 | 3 |
|---|---|---|---|---|---|---|---|---|
| Time after dose | Prior to dose | 30 min | 3h | 8h | 24h | 48h | 72h | Prior to dose |
| ID-38 | 2.2 | 2.1 | 2.3 | 4.2 | 3.4 | 3.1 | 2.1 | 3.4 |
| ID-39 | 3.6 | 3 | 2.4 | 6.1 | 5.2 | 5.4 | 4.1 | 3.5 |
| ID-40 | 11.6 | 8.8 | 9.4 | 11 | 12 | 11 | 8.9 | 8.3 |
|  | | | | | | | | |
| Dose | 13 | 15 | 15 | 15 | 15 | 15 | 15 | 15 |
| Time after dose | Prior to dose | Prior to dose | 30 min | 3h | 8h | 24h | 48h | 72h |
| ID-38 | 5.7 | 5.9 | 6.2 | 6 | 6.9 | 5.6 | 6.1 | 4.6 |
| ID-39 | 7.5 | 6.2 | 7.2 | 6.5 | 7.2 | 5.3 | 11.4 | 4.9 |
| ID-40 | 10.8 | 11.5 | 14.5 | 14.6 | 11.7 | 11.1 | 10.8 | 8.7 |

Subjects returned in 2 weeks (Day 28) for an end of substudy visit to assess safety. After the end of the substudy visit on Day 28, subjects returned to their IV rIX-FP prophyhlaxis regimen in the main study.

**Pharmacokinetic Analyses**

NONMEM® program Version 7.3 was used in this analysis (ICON Development Solutions, Ellicott City, Maryland, USA) [NONMEM user guide]. Pirana (version 2.8.1) was used as the NONMEM interface. PK parameters were estimated using the First-Order Conditional Estimation Method with Interaction (FOCEI). Perl-speaks-NONMEM (PsN, version 3.5.3) was used for NONMEM execution and VPC. R (version 2.14.1) (http://r-project.org) was used for post-processing and plotting of results.

The previously developed intravenously administered rIX-FP (2-compartment) population PK model for FIX activity from CSL654 (Idelvion final model) using data from Studies CSL654_2001, CSL654_2004, CSL654_3001, CSL654_3002 was used as a starting point for the present analysis. The disposition parameters were expressed in terms of clearance and volume of distribution parameters. The previous modeling has indicated that it may be advantageous to use log-transformed data; hence, log-transformed FIX activity were explored. Simultaneous fitting of FIX activity following intravenous and subcutaneous routes of rIX-FP administration was implemented using the Advan4 Trans4 model library within NONMEM.

For the FIX activity PK models, the true endogenous FIX activity levels were assumed not greater than 2% FIX activity to be consistent with the study inclusion criteria of FIX activity $\leq 2\%$. Therefore, where subjects had an observed FIX activity value greater than 2% prior to the first dose of rIX-FP, it was assumed that this reflected the residual contribution of the previous FIX product in addition to the true endogenous FIX activity levels. Therefore, the observed FIX activity levels (FTOT) were assumed to be the sum of the endogenous baseline levels (FEND), any residual contribution of the previous FIX product (FPP) and the increase in FIX levels attributed to CSL654 administration (FEX), as shown in the equation below:

$$FTOT = FEX + \mathrm{FEND} + \mathrm{FPP}$$

where:

FPP=BSE*EXP(-(CL/V1)*TIME) but if BSE < 2 then FPP=0.
BSE = the observed predose FIX activity
When FPP declines below the lower of the following then FPP=0: BSE, screening FIX activity, or 2%.
**Covariate modeling:** Body weight was considered as the significant covariate in this analysis.

**Model Evaluation:** The goodness-of-fit (GoF) for a model was assessed by a variety of plots and computed metrics:

• Observed versus population and individual predicted concentration plots;
• Conditional weighted residuals (CWRES) versus population predicted concentrations and versus time plots;

The final model was a 2 compartment model with simultaneous fitting of SC data from Cohort 1 and 3 of the 3003 study and IV data from the studies including 2001, 2004, 3001, 3002 and 3003. Body weight and weight adjusted dose were significant covariates on central volume and body weight was significant covariate on clearance.

**Simulations:**

The final FIX activity population PK model was used to simulate FIX activity-time profiles (both FTOT and FEX) for the following IV dosing scenarios in adolescent/adult populations:

• Steady state dose of 25 IU/kg every day
• Steady state dose of 25 IU/kg every 2 days
• Steady state dose of 25 IU/kg every 3 days

For these simulations, the individual observed predose FIX activity levels and individual body weights from the adolescent/adult included in the population PK modeling were used in the simulation datasets, and a 10-minute infusion duration was assumed. For each simulation scenario, 1000 replicates of the simulation datasets were performed.

The simulated and observed trough FIX activity levels are summarized in Table 10.

**Table 10: Simulated and observed trough FIX activity levels (IU/dL)**

| Regimen | Simulated Trough FIX activity (IU/dL) Median (95% CI) | Observed Trough FIX activity (IU/dL) 3003 Study - prior to 15th dose Cohort 3 (n=3) Median (min-max) |
|---------|---------|---------|
| 25 IU/kg Every day | 17.09 (7.94 - 34.036) | NA |
| 25 IU/kg Every 2 days | 8.24 (3.86 - 16.86) | NA |
| 25 IU/kg Every 3 days | 5.32 (2.58 - 11.02) | 6.2 (5.9-11.5) |

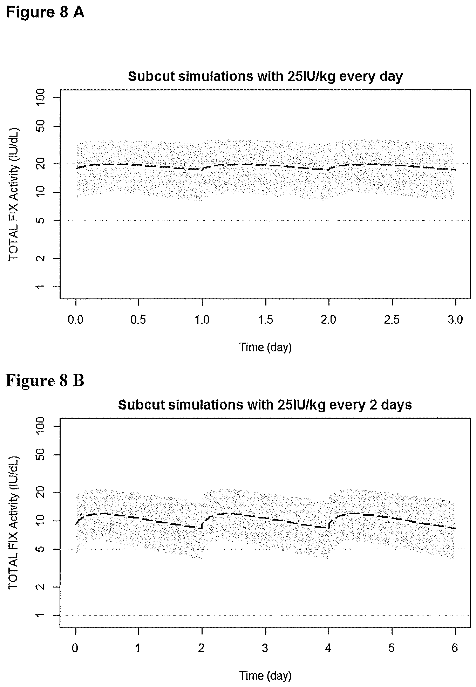

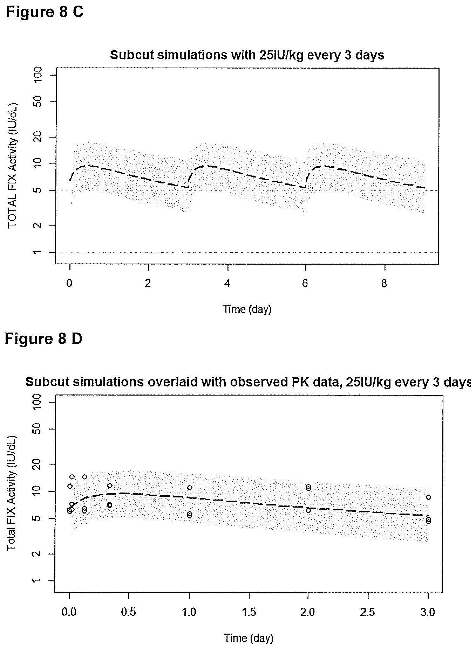

The simulated plot of FIX activity (IU/dL) vs Time for 25 IU/kg at time intervals of every day, every two days and every three days are shown in Figure 8A-C and the simulated plot of FIX Activity (IU/dL) vs Time for 25 IU/kg every 3 days overlaid with observed repeat PK data at 15th dose from cohort 3 (n=3) is shown in Figure 8D.

**Summary:**

The observed FIX activity data (median 6.2 IU/dL) following 25 IU/kg every 3 days, repeat subcutaneous rIX-FP dosing demonstrated the ability of this route of rIX-FP administration to maintain patient FIX activity levels within the mild hemophilia patient subcategory (e.g., >5 IU/dL FIX level). The revised population FIX PK model included and well-characterized the subcutaneous route of rIX-FP administration. The individual patient FIX PK fits following single and repeat subcutaneous dosing of rIX-FP demonstrate the utility of the final model. Simulations confirmed that the observed subcutaneous rIX-FP PK data was well aligned with the predicted PK profiles.

**Claims**

1. A fusion protein comprising

   a) human Factor IX (FIX), and
   b) human albumin

   for use in a method of preventing bleeding in a human patient in a prophylactic dosing regimen, wherein the human FIX is connected to the N-terminus of human albumin via a peptide linker cleavable by proteases involved in coagulation or activated by coagulation enzymes, and wherein the fusion protein is to be administered subcutaneously to the subject at a dose of about 10-50 IU/kg, using a dosing interval of about once a day to about once per week.

2. The fusion protein for use as in claim 1, wherein the dose is about 10 IU/kg, or about 25 IU/kg, or about 50 IU/kg.

3. The fusion protein for use as in claim 1 or 2, wherein the dosing interval is about once a day, or about four times a week, or about every 2 days, or about every 3 days, or about twice a week, or about every 5 days, or about once per week.

4. The fusion protein for use as in claims 1 to 3, wherein the dose is about 25 IU/kg, the dosing interval is about once a day and the total FIX activity trough level in the plasma is maintained between about 8 to about 34 IU/dL.

5. The fusion protein for use as in any one of claims 1 to 4, wherein the plasma level of the FIX maintains a trough level of at least about 1%, or at least about 3%, or at least about 5% or at least about 10% or at least about 15% above baseline for the entire dosing interval.

6. The fusion protein for use as in any one of claims 1 to 5, wherein the linker is cleavable by FIXa and/or by FVIIa/Tissue Factor (TF).

7. The fusion protein for use as in claims 6, wherein the sequence of the fusion protein has at least 70% identity to the sequence set forth in SEQ ID NO: 3.

8. The fusion protein for use as in claims 6 or 7, wherein the sequence of the fusion protein has the sequence set forth in SEQ ID NO: 3.

9. The fusion protein for use as in any one of claims 1 to 8, wherein the human patient suffers from hemophilia B.

## Patentansprüche

1. Fusionsprotein, umfassend

   a) menschlichen Faktor IX (FIX), und
   b) Humanalbumin,

   zur Verwendung bei einem Verfahren zur Vorbeugung von Blutungen bei einem menschlichen Patienten in einem prophylaktischen Dosierungsplan,
   wobei der menschliche FIX mit dem N-Terminus von Humanalbumin über einen durch Proteasen, die an der Blutgerinnung beteiligt sind oder durch Gerinnungsenzyme aktiviert werden, spaltbaren Peptidlinker verbunden ist und wobei das Fusionsprotein dem Individuum subkutan in einer Dosis von etwa 10-50 IU/kg zu verabreichen ist, wobei ein Dosierungsintervall von etwa einmal pro Tag bis etwa einmal pro Woche verwendet wird.

2. Fusionsprotein zur Verwendung nach Anspruch 1, wobei die Dosis bei etwa 10 IU/kg oder etwa 25 IU/kg oder etwa 50 IU/kg liegt.

3. Fusionsprotein zur Verwendung nach Anspruch 1 oder 2, wobei das Dosierungsintervall bei etwa einmal pro Tag oder etwa viermal pro Woche oder etwa alle 2 Tage oder etwa alle 3 Tage oder etwa zweimal pro Woche oder etwa alle 5 Tage oder etwa einmal pro Woche liegt.

4. Fusionsprotein zur Verwendung nach Anspruch 1 bis 3, wobei die Dosis bei etwa 25 IU/kg, das Dosierungsintervall bei etwa einmal am Tag liegt und der Talspiegel der FIX-Gesamtaktivität im Plasma zwischen etwa 8 bis etwa 34 IU/dl gehalten wird.

5. Fusionsprotein zur Verwendung nach einem der Ansprüche 1 bis 4, wobei der Plasmaspiegel des FIX einen Talspiegel von wenigstens etwa 1% oder wenigstens etwa 3% oder wenigstens etwa 5% oder wenigstens etwa 10% oder wenigstens etwa 15% über dem Grundwert für das gesamte Dosierungsintervall aufrecht erhält.

6. Fusionsprotein zur Verwendung nach einem der Ansprüche 1 bis 5, wobei der Linker durch FIXa und/oder durch FVIIa/Tissue Factor (TF) spaltbar ist.

7. Fusionsprotein zur Verwendung nach Anspruch 6, wobei die Sequenz des Fusionsproteins eine Identität von wenigstens 70% mit der Sequenz gemäß SEQ ID NO: 3 aufweist.

8. Fusionsprotein zur Verwendung nach Anspruch 6 oder 7, wobei die Sequenz des Fusionsproteins die Sequenz gemäß SEQ ID NO: 3 aufweist.

9. Fusionsprotein zur Verwendung nach einem der Ansprüche 1 bis 8, wobei der menschliche Patient an Hämophilie B leidet.

## Revendications

1. Protéine de fusion comprenant

   a) le facteur IX humain (FIX), et
   b) l'albumine humaine

   pour utilisation dans un procédé de prévention du saignement chez un patient humain dans un régime posologique prophylactique, le FIX humain étant relié à l'extrémité N-terminale de l'albumine humaine par l'intermédiaire d'un lieur peptidique clivable par des protéases impliquées dans la coagulation ou activées par des enzymes de coagulation, et la protéine de fusion étant destinée à être administrée par voie sous-cutanée au sujet à une dose d'environ 10 à 50 UI/kg, au moyen d'un intervalle d'administration d'environ une fois par jour à environ une fois par semaine.

2. Protéine de fusion pour utilisation selon la revendication 1, la dose étant d'environ 10 UI/kg, ou environ 25 UI/kg, ou environ 50 UI/kg.

3. Protéine de fusion pour utilisation selon la revendication 1 ou 2, l'intervalle d'administration étant environ une fois par jour, ou environ quatre fois par semaine, ou environ tous les 2 jours, ou environ tous les 3 jours, ou environ deux fois par semaine, ou environ tous les 5 jours, ou environ une fois par semaine.

4. Protéine de fusion pour utilisation selon les revendications 1 à 3, la dose étant d'environ 25 UI/kg, l'intervalle d'administration étant environ une fois par jour et le taux de vallée d'activité FIX dans le plasma étant maintenu entre environ 8 et environ 34 UI/dL.

5. Protéine de fusion pour utilisation selon l'une quelconque des revendications 1 à 4, le taux plasmatique du FIX maintenant un taux de vallée d'au moins environ 1 %, ou au moins environ 3 %, ou au moins environ 5 % ou au moins environ 10 % ou au moins environ 15 % au-dessus de la ligne de base pendant l'intervalle d'administration entier.

6. Protéine de fusion pour utilisation selon l'une quelconque des revendications 1 à 5, dans laquelle le lieur est clivable par FIXa et/ou par FVIIa/facteur tissulaire (TF).

7. Protéine de fusion pour utilisation selon les revendications 6, la séquence de la protéine de fusion ayant au moins 70 % d'identité avec la séquence décrite dans SEQ ID NO : 3.

8. Protéine de fusion pour utilisation selon les revendications 6 ou 7, la séquence de la protéine de fusion ayant la séquence décrite dans SEQ ID NO : 3.

9. Protéine de fusion pour utilisation selon l'une quelconque des revendications 1 à 8, le patient humain souffrant d'hémophilie B.

**Figure 1**

Figure 2

Figure 3

**Figure 4**

**Figure 5 A**

**Cohort 1 – 25 IU/kg**

**Figure 5 B**

**Cohort 2 – 50 IU/kg**

**Figure 6 A**

**Cohort 1 – 25 IU/kg**

**Figure 6 B**

**Cohort 2 – 50 IU/kg**

Figure 7

## Figure 8 A

Subcut simulations with 25IU/kg every day

## Figure 8 B

Subcut simulations with 25IU/kg every 2 days

## Figure 8 C

Subcut simulations with 25IU/kg every 3 days

## Figure 8 D

Subcut simulations overlaid with observed PK data, 25IU/kg every 3 days

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5925739 A
- WO 2012006624 A
- WO 2014052490 A
- WO 2015095925 A
- US 2008260755 A1

### Non-patent literature cited in the description

- *Thromb Haemost,* 1997, vol. 77 (5), 944-8
- GUIDELINES FOR THE MANAGEMENT OF HEMO-PHILIA
- **WHITE GC et al.** *Thromb Haemost.,* 1997, vol. 78, 261-265
- **EWENSTEIN BM et al.** Pharmacokinetic analysis of plasma-derived and recombinant F IX concentrates in previously treated patients with moderate or severe hemophilia B. *Transfusion,* 2002, vol. 42, 190-197
- **R.G. DI SCIPIO et al.** *Biochem.,* 1977, vol. 16, 6698-706
- **C. CHAUDHURY et al.** *J. Exp. Med.,* 2003, vol. 197 (3), 315-322
- Remington's Pharmaceutical Sciences